(19) Europäisches Patentamt / European Patent Office / Office européen des brevets

(11) **EP 3 875 118 A1**

(12) **EUROPEAN PATENT APPLICATION**
published in accordance with Art. 153(4) EPC

(43) Date of publication:
**08.09.2021 Bulletin 2021/36**

(21) Application number: **20891424.2**

(22) Date of filing: **17.12.2020**

(51) Int Cl.:
$A61K\ 47/68^{(2017.01)}$   $A61K\ 39/395^{(2006.01)}$
$A61P\ 3/10^{(2006.01)}$   $A61P\ 9/00^{(2006.01)}$
$A61P\ 9/10^{(2006.01)}$   $A61P\ 17/02^{(2006.01)}$
$A61P\ 17/06^{(2006.01)}$   $A61P\ 25/00^{(2006.01)}$
$A61P\ 25/08^{(2006.01)}$   $A61P\ 27/02^{(2006.01)}$
$A61P\ 27/06^{(2006.01)}$   $A61P\ 29/00^{(2006.01)}$
$A61P\ 35/00^{(2006.01)}$   $C07K\ 16/18^{(2006.01)}$

(86) International application number:
**PCT/JP2020/047157**

(87) International publication number:
**WO 2021/125263 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2019 JP 2019228542**

(71) Applicants:
• **National University Corporation Okayama University**
 **Okayama-shi, Okayama 700-8530 (JP)**
• **Sowakai Medical Foundation**
 **Kurashiki-shi, Okayama 710-0051 (JP)**

(72) Inventor: **The designation of the inventor has not yet been filed**

(74) Representative: **HGF**
 **1 City Walk**
 **Leeds LS11 9DX (GB)**

(54) **USE OF ANTIBODY-DRUG CONJUGATES AND ANTIBODIES FOR DRUG DELIVERY**

(57) One purpose of the present invention aims to provide a novel drug delivery means for targeting neovascularity. Another purpose of the invention is to provide a novel drug delivery means for targeting hyperpermeable vasculature. Still another purpose of the invention is to provide a method and a detection reagent for treatment or diagnosis of various diseases including malignant tumors.

The present conjugate of a drug and an anti-HMGB1 antibody or an antigen-binding fragment thereof is administered to a subject and incorporated into proangiogenic or hyperpermeable vascular endothelial cells to deliver the drug to the cells. In addition, the conjugate is used to provide a method and a detection reagent for treatment or diagnosis of various diseases including malignant tumors.

Fig. 2A

Anti-HMGB1 antibody (#10-22)

Anti-KLH antibody (control antibody)

1h   2h   4h (Time)

EP 3 875 118 A1

Printed by Jouve, 75001 PARIS (FR)

**Description**

TECHNICAL FIELD

The present invention relates to an antibody-drug conjugate using an anti-HMGB1 antibody and use of an anti-HMGB1 antibody or an antigen-binding fragment thereof for drug delivery.

BACKGROUND ART

In recent years, to treat cancer and/or other diseases, there have been developed molecular target drugs, including antibodies and antibody-drug conjugates that target specific molecules in diseased cells. However, it has been known that in cancer, for instance, mutations thereof cause tolerance or resistance to antibodies. In addition, since different cancers present different kinds of antigens, a specific antibody is applicable to limited types of cancers.
In view of the above, for solid tumors, for instance, there have been developed antibodies (e.g., VEGF antibodies) that target tumor angiogenic vasculatures rather than tumor cells themselves. In addition, immune checkpoint inhibitors (e.g., an anti-PD-1 antibody, an anti-CTLA-4 antibody) that unlock the suppression of innate immune response to tumors have been developed as drugs widely applicable to various tumors. However, antibodies that target neovascularity may inhibit not only tumor vasculatures but also normal angiogenesis. It is also known that the immune checkpoint inhibitors are actually effective only in some cancers.
Thus, an antitumor drug for treating a tumor by a new mechanism has been sought.
Meanwhile, disruption of the blood-brain barrier (BBB) has attracted attention as a phenomenon where a dynamic vasculature change occurs like in cancer. HMGB1 (High Mobility Group Box 1, Non-Patent Document 1), which is among endogenous damage-associated molecular patterns (DAMPs), is released extracellularly from cell nuclei of, for instance, nerve cells due to cerebral infarction, stroke, traumatic brain injury, or brain inflammation. Then, HMGB1 triggers an increase in inflammatory cytokines in the brain and increases cerebrovascular blood-brain barrier permeability. This results in the BBB disruption. The BBB disruption and inflammation cause brain edema and hypoxia, and inflammation promotes further release of HMGB1, resulting in positive feedback to make the symptoms worsen. Furthermore, it has been suggested that HMGB1 and the BBB disruption are also involved in epilepsy (Non-Patent Documents 2 and 3).
For instance, Patent Document 1 describes use of an anti-HMGB1 antibody or an antigen-binding fragment thereof as a neutralizing antibody for treatment or prophylaxis of various inflammatory diseases. However, in such a technology, anti-HMGB1 antibodies or antigen-binding fragments thereof in themselves are used to treat or prevent various inflammatory diseases.

PRIOR ART DOCUMENTS

PATENT DOCUMENT

Patent Document 1: WO2014/115430

NON-PATENT DOCUMENT

Non-Patent Document 1: Annual Review of Immunology, 2011, Vol. 29, pp. 139-162
Non-Patent Document 2: Nihon Yakurigaku Zasshi, 2018, Vol. 151, pp. 4-8
Non-Patent Document 3: Journal of Pharmacological Science, 2019, Vol. 140, pp. 94-101

SUMMARY OF THE INVENTION

PROBLEMS TO BE SOLVED BY THE INVENTION

In light of the above circumstances, one purpose of the present invention is to provide a novel drug delivery means for targeting neovascularity.
In light of the above circumstances, another purpose of the invention is to provide a novel drug delivery means for targeting hyperpermeable vasculature.
Still another purpose of the invention is to provide a method and a detection reagent for treatment or diagnosis of various diseases including malignant tumors.

MEANS FOR SOLVING THE PROBLEMS

The present inventors have conducted intensive research and, as a result, have found that a conjugate of a drug and an anti-HMGBI antibody or an antigen-binding fragment thereof can be taken up by proangiogenic vascular endothelial cells, and the drug can be delivered into the cells.

Further, the present inventors have found that the conjugate of a drug and an anti-HMGB1 antibody or an antigen-binding fragment thereof can be taken up by hyperpermeable vascular endothelial cells such as brain vasculatures in epilepsy.

Furthermore, the present inventors have also found that the conjugate of a drug and an anti-HMGB1 antibody or an antigen-binding fragment thereof is not substantially incorporated into normal vasculatures.

Based on the above findings, the present inventors have completed the invention.

Specifically, the invention provides the following antibody-drug conjugates.

[1] An antibody-drug conjugate comprising: an antibody specifically binding to HMGB1 or an antigen-binding fragment thereof; and a drug moiety conjugated to the antibody or the antigen-binding fragment thereof.

[2] The antibody-drug conjugate according to [1], wherein the drug moiety and the antibody or the antigen-binding fragment thereof are conjugated via at least one linker.

[3] The antibody-drug conjugate according to [1] or [2], wherein the drug moiety comprises one or two or more members selected from the group consisting of cytotoxic agents, cytoprotectants, probes, angiogenesis inhibitors, immunosuppressants, antihypertensive drugs, vasopressors, pain relievers, cytokines, antibiotics, and immune checkpoint inhibitors.

[4] The antibody-drug conjugate according to any one of [1] to [3], which is represented by formula (I):

$$A\text{-}(L\text{-}(D)_m)_n \qquad (I)$$

wherein A is the antibody specifically binding to HMGB1 or the antigen-binding fragment thereof;
L is a linker;
D is the drug moiety;
m is an integer of 1 to 8; and
n is an integer of 1 to 20.

[5] The antibody-drug conjugate according to any one of [1] to [4], wherein the antibody or the antigen-binding fragment thereof specifically binds to human HMGB1 and is a chimeric antibody, a humanized antibody, or a human antibody, or a fragment thereof.

[6] The antibody-drug conjugate according to any one of [1] to [5], wherein the antibody is a monoclonal antibody.

[7] The antibody-drug conjugate according to any one of [1] to [6], wherein the antibody or the antigen-binding fragment thereof specifically binds to a human HMGB1 epitope comprising an amino acid sequence set forth in SEQ ID NO: 1 or SEQ ID NO: 2.

[8] The antibody-drug conjugate according to any one of [2] to [7], wherein the linker is selected from the group consisting of cleavable linkers, non-cleavable linkers, hydrophilic linkers, procharge linkers, and dicarboxylic acid-based linkers.

Also, the invention provides the following pharmaceutical compositions.

[9] A pharmaceutical composition comprising the antibody-drug conjugate according to any one of [1] to [8] and a pharmaceutically acceptable carrier.

[10] The pharmaceutical composition according to [9], which is used for treatment of a proangiogenic disease or a vascular hyperpermeability-related disease.

[11] The pharmaceutical composition according to [9] or [10], which is used for treatment of:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or
(b) epilepsy, stroke, cerebral infarction, cerebral vasospasm, traumatic brain injury, chronic inflammation, neurodegenerative diseases, acute inflammation, or sepsis.

[12] The pharmaceutical composition according to any one of [9] to [11], which is used in combination with one or two or more members selected from the group consisting of other antibodies, other antibody-drug conjugates, cytotoxic agents, cytoprotectants, probes, angiogenesis inhibitors, immunosuppressants, antihypertensive drugs, vasopressors, pain relievers, cytokines, antibiotics, and immune checkpoint inhibitors.

In addition, the invention provides the following detection reagents:

[13] A detection reagent comprising the antibody-drug conjugate according to any one of [1] to [8].
[14] The detection reagent according to [13], wherein the drug moiety comprises at least a probe.

Further, the invention provides the following use:

[15] Use of an antibody specifically binding to HMGB1 or an antigen-binding fragment thereof for drug delivery to a cell.
[16] The use according to [15], wherein the cell is a vascular endothelial cell.
[17] The use according to [15] or [16], comprising treating the cell with the antibody-drug conjugate according to any one of [1] to [8].

Furthermore, the invention provides the following methods for treatment or diagnosis:

[18] A method for treating or diagnosing a proangiogenic disease or a vascular hyperpermeability-related disease, said method comprising administering, to a subject in need thereof, the antibody-drug conjugate according to any one of [1] to [8].
[19] A method for treating or diagnosing:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or
(b) epilepsy, stroke, cerebral infarction, cerebral vasospasm, traumatic brain injury, chronic inflammation, neurodegenerative diseases, acute inflammation, or sepsis;

said method comprising administering, to a subject in need thereof, the antibody-drug conjugate according to any one of [1] to [8].
[20] The method according to [18] or [19], further comprising administering, to the subject, one or two or more members selected from the group consisting of other antibodies, other antibody-drug conjugates, cytotoxic agents, cytoprotectants, probes, angiogenesis inhibitors, immunosuppressants, antihypertensive drugs, vasopressors, pain relievers, cytokines, antibiotics, and immune checkpoint inhibitors.
[21] The method according to any one of [18] to [20], further comprising irradiating the subject with a proton beam, a heavy particle beam, a gamma ray, an X-ray, or a light.

Furthermore, the invention includes the following antibody-drug conjugates:

[22] The antibody-drug conjugate according to any one of [1] to [8] for use in treatment or diagnosis of proangiogenic diseases or vascular hyperpermeability-related diseases.
[23] The antibody-drug conjugate according to any one of [1] to [8] for use in treatment or diagnosis of:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or
(b) epilepsy, stroke, cerebral infarction, cerebral vasospasm, traumatic brain injury, chronic inflammation, neurodegenerative diseases, acute inflammation, or sepsis.

Moreover, the invention includes the following use for the manufacture:

[24] Use of the antibody-drug conjugate according to any one of [1] to [8] for the manufacture of a pharmaceutical composition for treatment or diagnosis of proangiogenic diseases or vascular hyperpermeability-related diseases.
[25] Use of the antibody-drug conjugate according to any one of [1] to [8] for the manufacture of a pharmaceutical composition for treatment or diagnosis of:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or

(b) epilepsy, stroke, cerebral infarction, cerebral vasospasm, traumatic brain injury, chronic inflammation, neurodegenerative diseases, acute inflammation, or sepsis.

EFFECT OF THE INVENTION

According to the construction of the invention, a drug conjugated to an antibody or an antigen-binding fragment thereof is specifically delivered to proangiogenic or hyperpermeable vascular endothelial cells. Then, the delivered drug can act on the vascular endothelial cells to effectively prevent, improve, or treat proangiogenic diseases or vascular hyperpermeability-related diseases. In addition, the invention can provide a method and a detection reagent for treatment or diagnosis of various diseases including malignant tumors.

BRIEF DESCRIPTION OF THE DRAWINGS

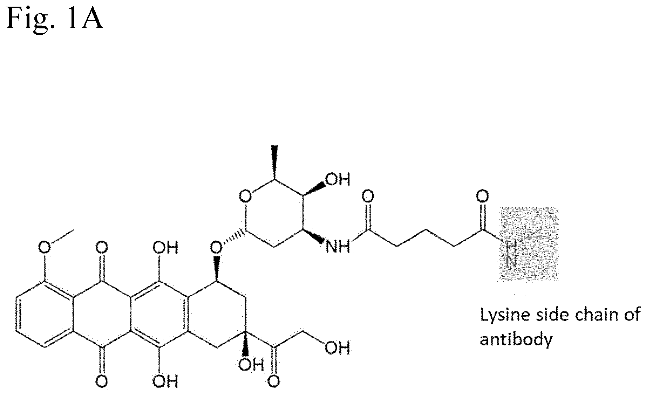

Fig. 1A is a chemical structural formula representing doxorubicin and its linker portion in a doxorubicin-conjugated antibody used in Examples. The shaded area corresponds to the amino group portion of a lysine side chain of the antibody.

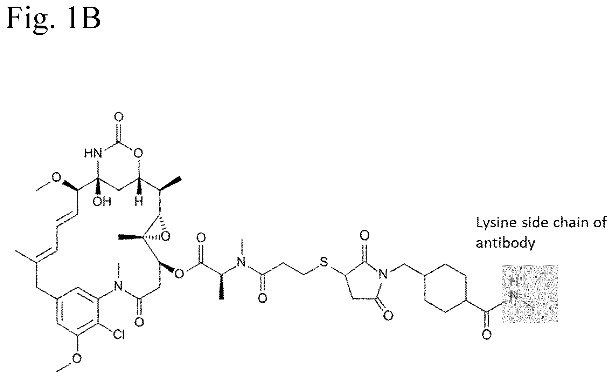

Fig. 1B is a chemical structural formula representing an emtansine moiety in an emtansine-conjugated antibody used in Examples. The shaded area corresponds to the amino group portion of a lysine side chain of the antibody.

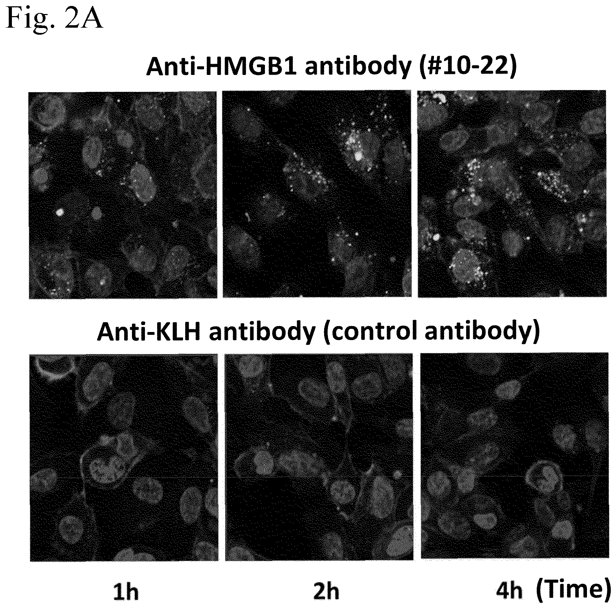

Fig. 2A represents photographs showing antibody localization in cultured vascular endothelial cells incubated with a fluorescently labeled anti-HMGBl antibody (#10-22) or anti-KLH antibody for varied times in Test Example 1. From the left, Fig. 2A shows images obtained at 1, 2, or 4 h after addition of each antibody. Fluorescently labeled antibody (Alexa Fluor 488) is shown in green, cell nuclei (DAPI) are shown in blue, and F-actin is shown in red.

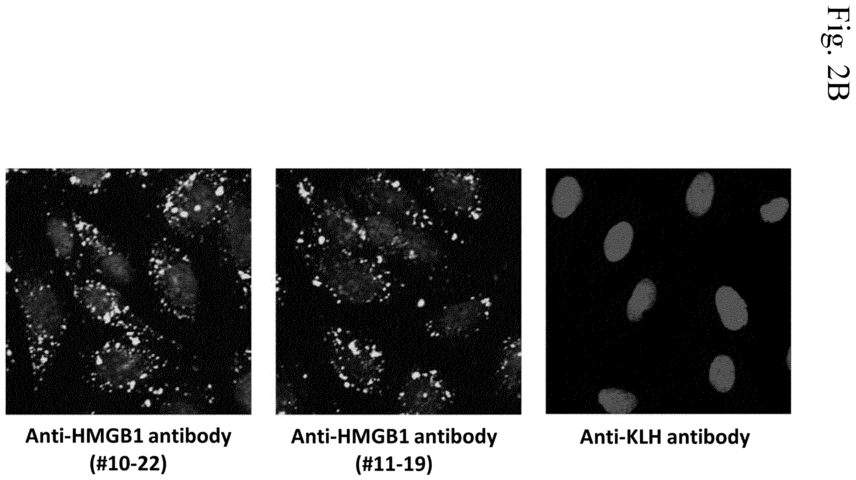

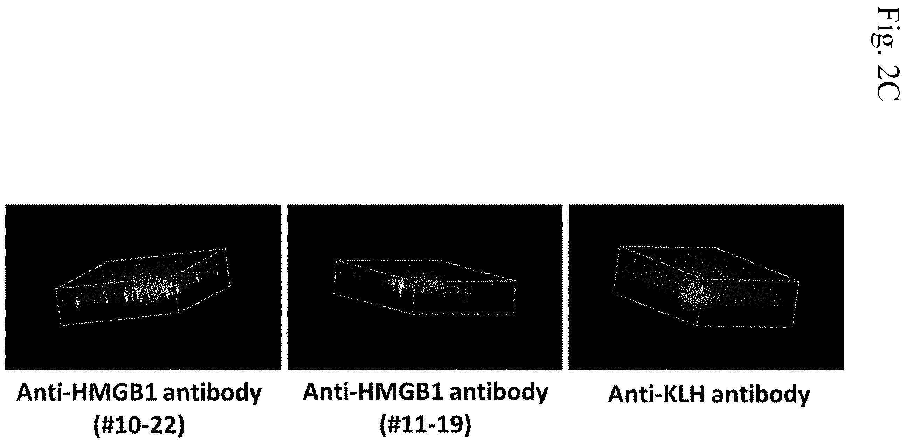

Figs. 2B and 2C are photographs showing antibody localization in cultured vascular endothelial cells incubated with a fluorescently labeled anti-HMGBl antibody (#10-22 or #11-19) or a fluorescently labeled anti-KLH antibody for 4 h in Test Example 1. Fig. 2B shows the two-dimensional localization, and Fig. 2C shows the three-dimensional localization. Fluorescently labeled antibody (Alexa Fluor 488) is shown in green and cell nuclei (DAPI) are shown in blue.

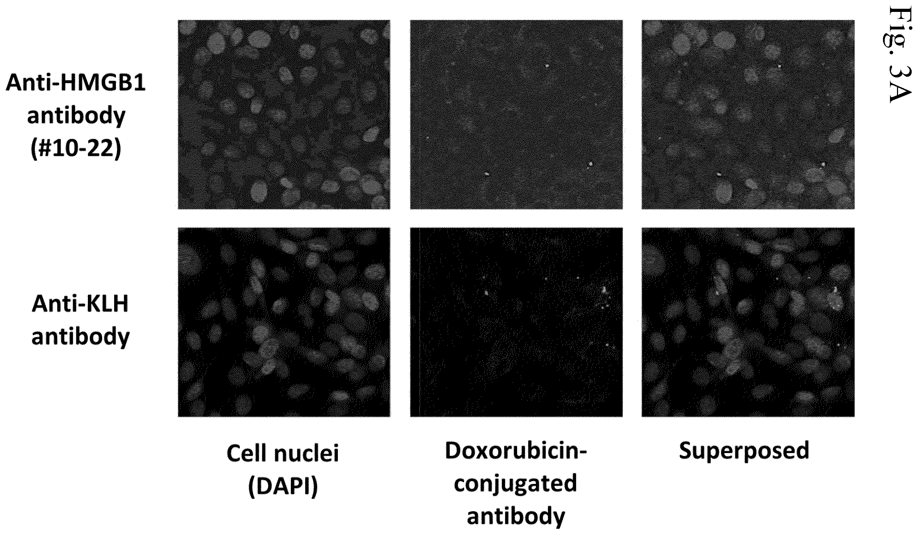

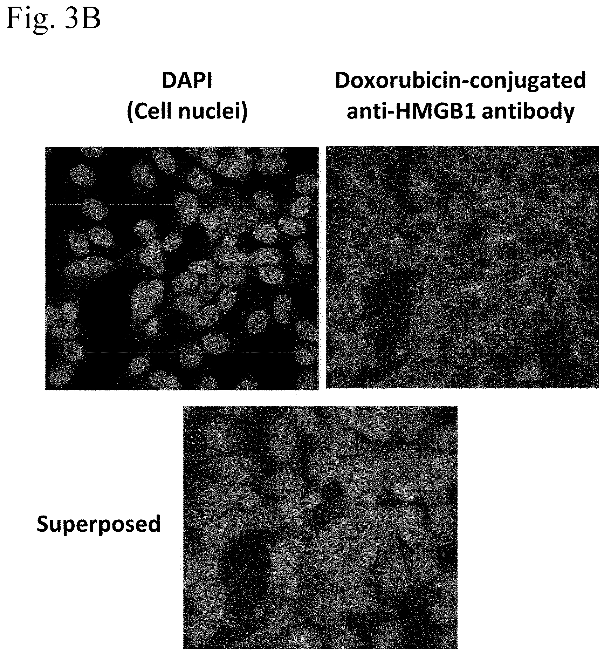

Figs. 3A and 3B are photographs showing antibody localization in cultured vascular endothelial cells incubated with a doxorubicin-conjugated anti-HMGBl antibody (#10-22) or a doxorubicin-conjugated anti-KLH antibody for 4 h in Test Example 2.

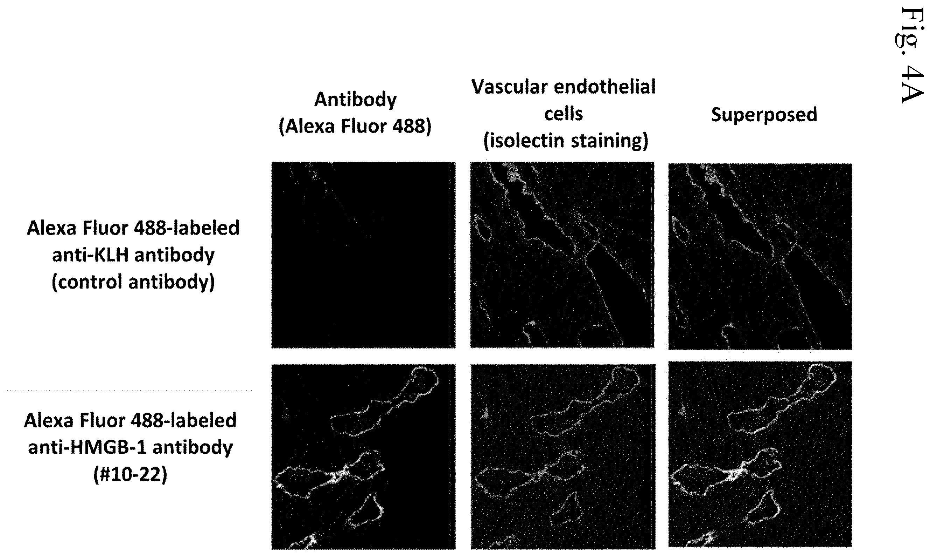

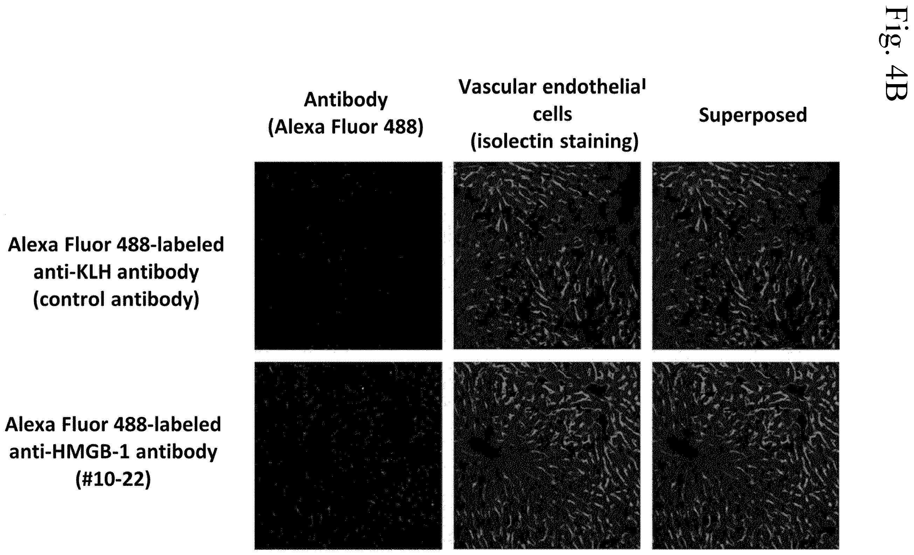

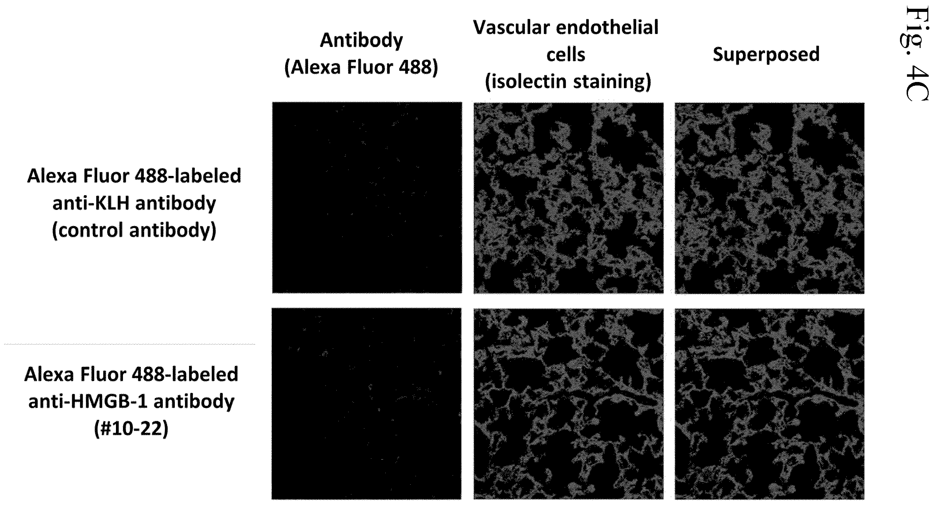

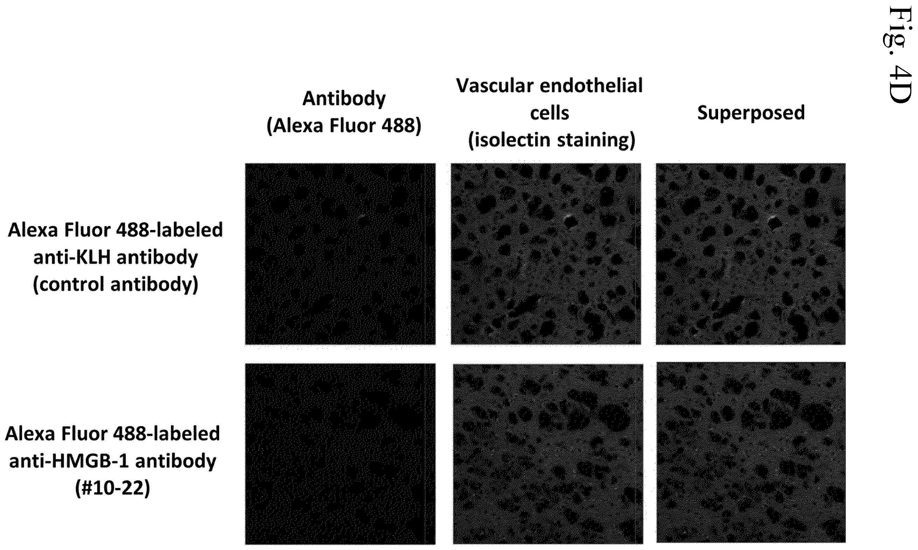

Figs. 4A to 4D are photographs showing the distribution of Alexa Fluor 488-labeled anti-HMGBl antibody administered to melanoma-bearing mice in Test Example 3. Fig. 4A shows the distribution in melanoma vasculature, Fig. 4B shows the distribution in the liver, Fig. 4C shows the distribution in the lung, and Fig. 4D shows the distribution in the brain.

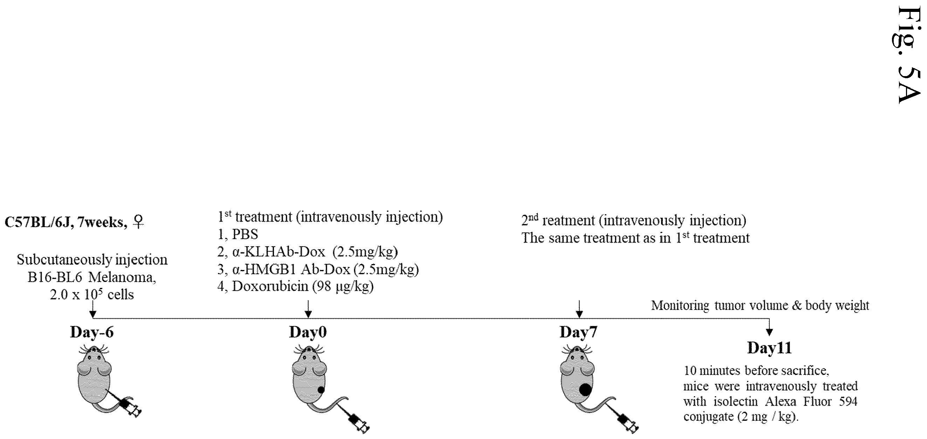

Fig. 5A is a diagram illustrating an overview of the test schedule in Test Example 4.

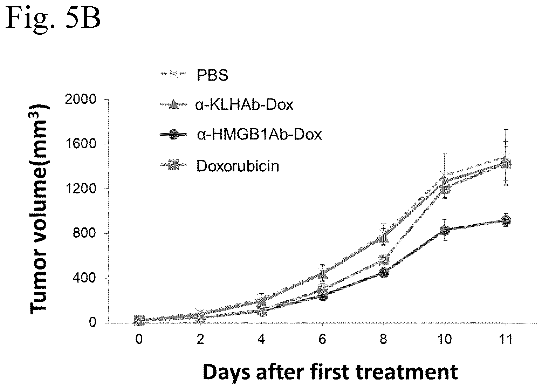

Fig. 5B is a graph showing changes in the tumor volume of mice in each treatment group (n = 4) from the start of drug administration to day 11 in Test Example 4. An unpaired t-test was used as the test between each group. The two-sided p value between the $\alpha$-HMGB1Ab-Dox group and the PBS, $\alpha$-KLHAb-Dox, or Doxorubicin group was 0.047, 0.047, or 0.021, respectively.

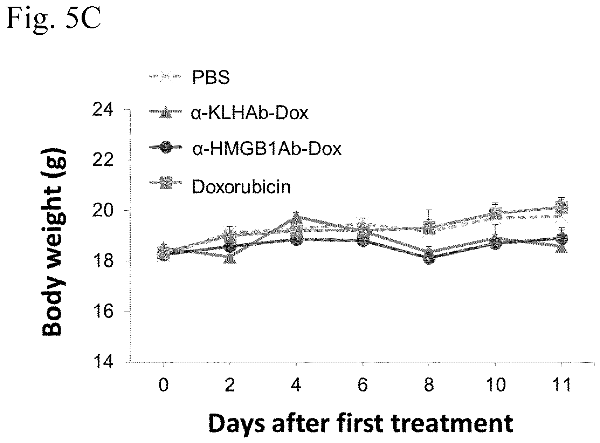

Fig. 5C is a graph showing changes in the body weight of mice in each treatment group (n = 4) from the start of drug administration to day 11 in Test Example 4. Student t-test was used for the test between each group. Any of the two-sided p-value between the $\alpha$-HMGB1Ab-Dox group and the PBS, $\alpha$-KLHAb-Dox, or Doxorubicin group was greater than 0.05.

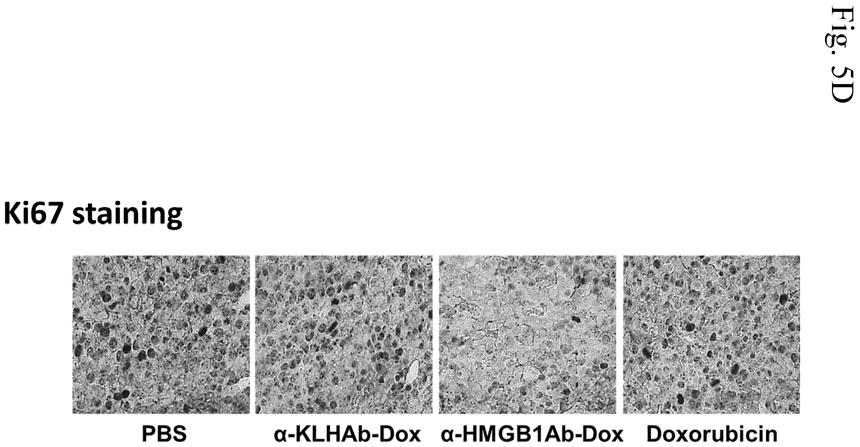

Fig. 5D is Ki67 staining images of melanoma tissue sections obtained from mice in each group at day 11 after the drug administration in Test Example 4.

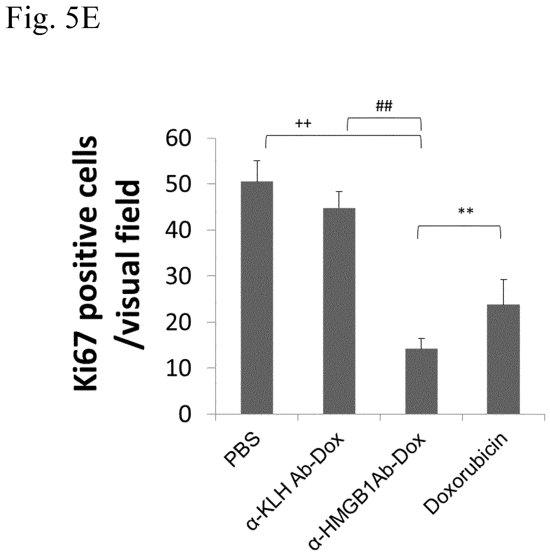

Fig. 5E is a graph comparing the number of Ki67-positive cells in Ki67 staining images of melanoma tissue sections obtained from mice in each group at day 11 after the drug administration in Test Example 4. The p-values were ++: p = 0.000029, ##: p = 0.00428, and *: p = 0.015, respectively. Student t-test was used for the test.

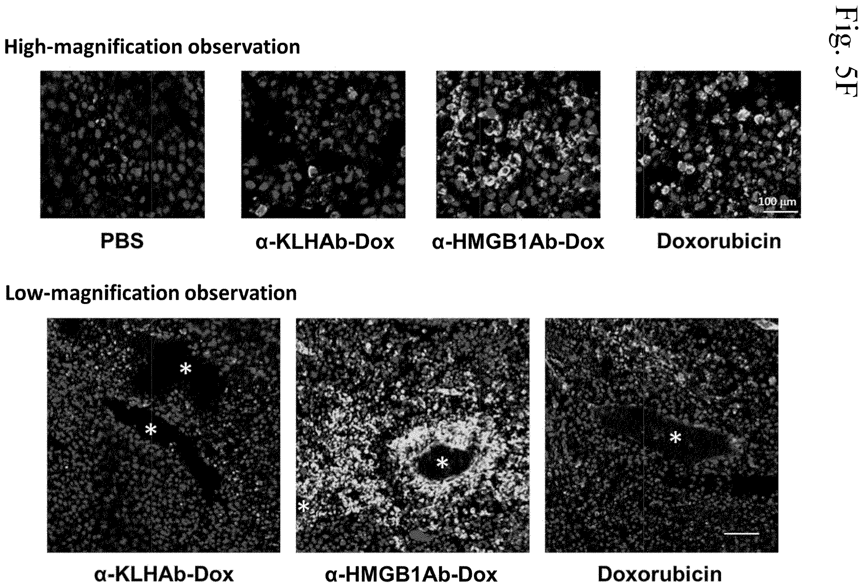

Fig. 5F is staining images of apoptotic cells in melanoma tissue sections obtained from mice in each group at day 11 after the drug administration in Test Example 4. Apoptotic cells (TUNEL staining) are shown in green, and cell nuclei (DAPI) are shown in blue. * in each low-magnification image denotes a vascular lumen.

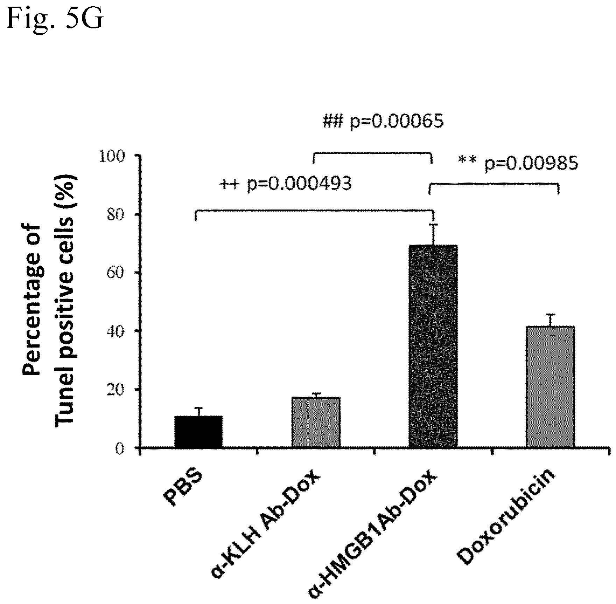

Fig. 5G is a graph comparing the percentage of apoptotic cells in melanoma tissue sections obtained from each group as counted from the TUNEL staining images in Test Example 4. All the p-values on the graph are values obtained by Student t-test.

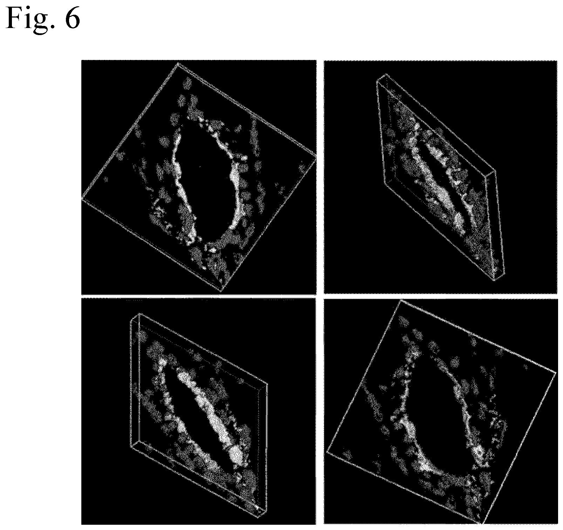

Fig. 6 represents photographs showing that a fluorescently labeled anti-HMGB1 antibody administered to and accumulated in brain vasculatures of a pilocarpine-induced status epilepticus (PILO-SE) model mouse in Test Example 5. Fluorescently labeled antibody (Alexa Fluor 488) is shown in green and cell nuclei (DAPI) are shown in blue.

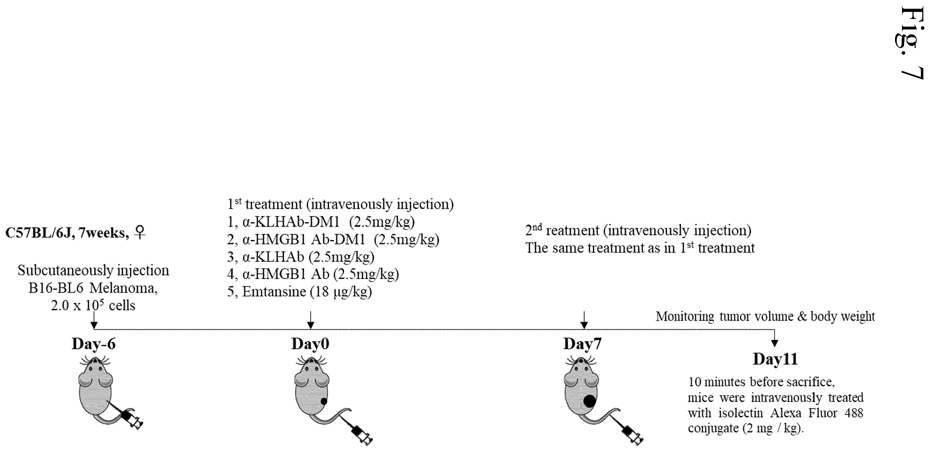

Fig. 7 is a diagram showing an overview of the test schedule in Test Example 6.

MODE FOR CARRYING OUT THE INVENTION

[Description of terms]

As used herein, scientific terms and technical terms used in connection with the invention each have the meaning generally understood by those skilled in the art. Further, unless otherwise indicated in some context, the singular term includes reference to the plural term and the plural term includes reference to the singular term. Generally speaking, the nomenclature used in connection with the cell and tissue culture, molecular biology, immunology, microbiology, genetics and protein chemistry, nucleic acid chemistry, and hybridization techniques described herein is well-known in the art and commonly used.

As used herein, HMGB1 represents the above-mentioned High Mobility Group Box 1 (see Non-Patent Document 1). HMGB1 is a non-histone chromosomal protein present in almost all eukaryotic cells and is one of the endogenous damage-associated molecular patterns (DAMPs). For example, human HMGB1 gene is a gene deposited as NCBI Gene ID: 3146 in NCBI (https://www.ncbi.nlm.nih.gov/). As used herein, isoforms of the HMGB1 protein are not particularly limited as long as the isoforms have the characteristics of HMGB1.

The HMGB1 protein is a 25 kDa protein consisting of 215 amino acids rich in lysine residues and has an amino acid sequence very highly conserved among mammals. Its structure includes three domains: two DNA-binding domains called A-box (or box-A) and B-box (or box-B); and a C-terminal region (also called a C-terminal domain or acidic tail) consisting of aspartic acid and glutamic acid moieties. A-box and B-box are each composed of about 80 highly conserved amino acid residues and are strongly positively charged. B-box has a TLR4 (toll-like receptor 4) binding domain and a RAGE (receptor for advanced glycation end products) binding domain. It is known that the amino acid sequence of the C-terminal region of HMGB1 protein is highly conserved, with only a few differences between mammals.

The HMGB1 protein is not only localized in the nucleus of a cell, but also translocates from the nucleus to the cytoplasm while macrophages and various immune system cells are activated, and is then secreted extracellularly (active secretion). In addition, as described above, it has been revealed that HMGB1 localized in the nucleus is rapidly released (passive release) due to cell necrosis or apoptosis caused by ischemia or injury. The released HMGB1 then acts as a powerful inflammatory mediator via TLR4, RAGE, etc., and causes an inflammatory immune response.

As used herein, the term "antibody" refers to a polypeptide of the immunoglobulin family capable of binding to an antigen non-covalently and specifically.

Of the naturally occurring immunoglobulin molecules, IgG consists of four polypeptide chains, i.e., two heavy chains and two light chains, interconnected by disulfide bonds. Each heavy chain consists of a heavy chain variable region (sometimes referred to as "VH") and a heavy chain constant region (the heavy chain constant region consists of three domains, which are each referred to as "CH1", "CH2", or "CH3"). Each light chain consists of a light chain variable region (sometimes referred to as "VL") and a light chain constant region (sometimes referred to as "CL") .

Meanwhile, VH and VL are important because they involve the binding specificity of antibody. Since antibodies interact with target antigens primarily through the amino acid residues of VH and VL, the amino acid sequence within the variable region is more different between individual antibodies than the sequence outside the variable region. In addition, VH and VL can be further divided into regions called framework regions (FRs), which are more conserved among various antibodies, and hypervariable regions called complementarity determining regions (CDRs). VH and VL each consist of 3 CDRs and 4 FRs, which are arranged from the amino terminus to the carboxy terminus in the order of FR1, CDR1, FR2, CDR2, FR3, CDR3, and FR4.

As used herein, the term "anti-HMGBI antibody" refers to an antibody specifically binding to HMGB1.

As used herein, the "complementarity determining regions" or "CDRs" are hypervariable regions of VL or VH that are involved in antigen specificity. As used herein, the heavy chain CDR1 to 3, which are CDRs of the heavy chain variable region, are sometimes referred to as CDRH1 to 3. In addition, as used herein, light chain CDRs 1 to 3, which are CDRs of the light chain variable region, are sometimes referred to as CDRL1 to 3.

As used herein, the positions of CDRs and framework regions are according to Kabat's definition. That is, the positions are defined based on Kabat, E.A., et al., Sequences of Proteins of Immunological Interest, 5th ed. Public Health Service, National Institutes of Health, Bethesda, MD (1991), NIH Publication 91-3242.

As used herein, the term "antigen-binding fragment" refers to an antibody fragment that retains an ability to specifically interact with an epitope in an antigen and functions to specifically interact with the antigen of the original antibody.

Examples of the antibody fragment include, but are not particularly limited to, a Fab fragment, a F(ab')$_2$ fragment, an Fd fragment consisting of VH and CH1 domains, an Fv fragment consisting of VL and VH domains of a single arm of an antibody, a dAb fragment consisting of a VH domain, isolated complementarity determining regions with frameworks allowing for specific binding, a bispecific antibody, or a multispecific antibody. These antibody fragments are reviewed in, for instance, Hudson, PJ et al., Nat. Med. 9 (2003) 129-134; Plueckthun, A., In: The Pharmacology of Monoclonal Antibodies, Vol. 113, Rosenburg and Moore (eds), Springer-Verlag, New York (1994), pp. 269-315; and Nature Vol.341, pp.544-546, 1989.

As used herein, the term "epitope" refers to a site on an antigen to which an antibody or antigen-binding fragment specifically binds.

As used herein, the term "human antibody" refers to an antibody having a variable region and a variable region in which both FRs and CDRs are derived from a human immunoglobulin.

As used herein, the term "chimeric antibody" refers to, for instance, an antibody obtained by combining a variable region derived from a non-human animal antibody and a constant region of a human antibody.

As used herein, the term "humanized antibody" refers to, for instance, an antibody in which variable region CDRs derived from a non-human animal antibody are grafted in a human antibody or an antibody in which some of CDRs and/or FRs derived from a non-human animal antibody are grafted in a human antibody.

The FRs of a human antibody or humanized antibody may contain amino acid residues that are not encoded by the human immunoglobulin gene due to, for instance, random mutations or site-specific mutations introduced *in vivo* or *in vitro.*

As used herein, the "antibody-drug conjugate" or "conjugate" is a product obtained by conjugating an antibody or an antigen-binding fragment thereof and a drug such as a cytotoxic agent, a cytoprotectant, or a probe other than the antibody or the fragment thereof.

As used herein, "conjugation" may involve covalent bonds or non-covalent bonds (examples include, but are not limited to, coordination bonds, hydrophobic bonds, hydrogen bonds, electrostatic bonds) or both.

In addition, the term "conjugated" is not limited to the case where a drug and an antibody or an antigen-binding fragment thereof that are originally separated become conjugated. That is, the "antibody-drug conjugate" includes, for instance, the case where the entire or part of a linker and a drug moiety is a polypeptide; and at least one polypeptide of the antibody or antigen-binding fragment and a polypeptide portion of the linker and a drug moiety are fused as one polypeptide.

As used herein, the term "cytotoxic agent" refers to a drug that is toxic to cell growth and proliferation and acts in such a manner of reducing the number of cells, inhibiting the proliferation of cells, or collapsing cells.

As used herein, the term "cell protectant" refers to a drug that is beneficial to cell growth and proliferation and acts in such a manner of increasing the number of cells, inhibiting cell death, repairing the structure of cell, or improving functions of cells.

As used herein, the term "probe" refers to a substance that can visualize the morphology or state of an organ, tissue, or cell, or part thereof. Preferably, the probe does not substantially function as a cytotoxic agent. More preferably, the probe functions as neither a cytotoxic agent nor a cytoprotectant essentially.

As used herein, the "linker" is any chemical part capable of linking an antibody or antibody fragment (e.g., an antigen-binding fragment) to another portion such as a drug moiety.

As used herein, the term "subject" refers to an individual (e.g., a human, non-human animal), organ, tissue, or cell. If the individual is a human, a "patient" is used interchangeably with a "subject" unless otherwise indicated.

As used herein, the term "treatment" or "treat" includes "medical treatment", "prevention", as well as drug exposure to non-individuals such as organs, tissues, or cells. The term "medical treatment" means to cure, improve or alleviate a disease, symptom, or health condition; or to reduce, prevent, or delay aggravation of a disease, symptom, or health condition; or to reverse, reduce, prevent, or delay progression of a disease or symptom.

As used herein, the term "prevention" means preventing the onset of a disease or symptom; or reducing the risk of developing a disease or symptom.

[Use of anti-HMGBI antibody or antigen-binding fragment thereof for drug delivery]

Use of the invention relates to use of an antibody specifically binding to HMGB1 (anti-HMGB1 antibody) or an antigen-binding fragment thereof for drug delivery to cells. The antibody specifically binding to HMGB1 or antigen-binding fragment thereof used may be the same as those described below in the section [Antibody-drug conjugate] .

In one embodiment, the cell to be used in the invention means a cell contained in, for instance, an *in vivo* organ or tissue or an organ or tissue separated from a living body, or an isolated, cultured, or differentiated cell.

In another embodiment, during use of the invention, a drug is delivered to vascular endothelial cells, preferably delivered specifically to proangiogenic or hyperpermeable vascular endothelial cells.

In still another embodiment, the above drug is conjugated to an anti-HMGB1 antibody or antigen-binding fragment thereof and taken up by the above cells while the drug is an antibody-drug conjugate. The antibody-drug conjugate used may be those described below in the section [Antibody-drug conjugate].

Although the mechanism is unknown, the drug-conjugated anti-HMGBI antibody or antigen-binding fragment thereof specifically accumulates in proangiogenic or hyperpermeable vascular endothelial cells and is incorporated into the cells. However, the antibody-drug conjugate does not substantially accumulate in normal vascular endothelial cells. In view of such characteristics, the invention exerts, for instance, the following effects or applications.

(1) The drug can be delivered highly efficiently to proangiogenic or hyperpermeable vascular tissues (e.g., tumors), and systemic adverse effects caused by the drug are thus eliminated or reduced.
(2) In one embodiment, the invention is applicable to diseases in which angiogenesis or vascular permeability is expected to be promoted.
(3) In another embodiment, use of a cytotoxic agent as the drug makes it possible to damage a blood vessel(s) in an undesirable in vivo lesion such as a tumor, and suppress the supply of nutrients and oxygen to the lesion.
(4) In still another embodiment, use of a cytoprotectant as the drug makes it possible to improve conditions and treat a disease related to vascular permeability, for instance, at the blood-brain barrier where vascular permeability is abnormally increased.
(5) In yet another embodiment, the invention may involve use to obtain information about the morphology or conditions of an individual, organ, tissue, or cell. For instance, use of a probe as the drug makes it possible to obtain information about the morphology and conditions of hyperpermeable vasculature and their cells.

[Antibody-drug conjugate]

An antibody-drug conjugate of the invention comprises an antibody specifically binding to HMGB1 (anti-HMGB1 antibody) or an antigen-binding fragment thereof, and one or more drug moieties conjugated to the antibody or the antigen-binding fragment thereof.

When a plurality of drugs are conjugated to the antibody-drug conjugate of the invention, each drug may be directly conjugated to the antibody. Alternatively, in the antibody-drug conjugate of the invention, one or more drugs may be indirectly conjugated via other drug(s) to the antibody or the antigen-binding fragment thereof.

In one embodiment, the antibody-drug conjugate of the invention comprises at least one linker through which the antibody or antigen-binding fragment thereof and a drug moiety are linked using covalent and/or non-covalent bond(s).

In another embodiment, an antibody-drug conjugate of the invention is represented by the following formula (I):

$$A\text{-}(L\text{-}(D)_m)_n \qquad (I)$$

wherein A is an antibody specifically binding to HMGB1 or an antigen-binding fragment thereof; L is a linker; D is a drug moiety; m is, for instance, an integer of 1 to 8; and n is an integer of 1 to 20.

More preferably, in the antibody-drug conjugate of formula (I) above, m is, for instance, an integer of 1 or more, and preferably an integer of 1, 2, 3 or 4; n is, for instance, an integer of 1 or more, and preferably an integer of 1 to 10, 2 to 8, or 2 to 5.

The drug/antibody ratio (DAR), which indicates the average number of drug molecules per antibody molecule in an antibody-drug conjugate of the invention, is not particularly limited and may be selected, if appropriate, depending on, for instance, the type of drug used and/or the application target. From the viewpoint of enhancing antitumor activity, the DAR of an antibody-drug conjugate of the invention is, for instance, 1 or larger, or 2 or larger, preferably 3 or larger, more preferably 4 or larger, still more preferably 5 or larger, and still more preferably 6 or larger. In addition, from the viewpoint of suppressing adverse effects, the DAR per antibody molecule in an antibody-drug conjugate of the invention is, for instance, 15 or less, 12 or less, 10 or less, 9 or less, 8 or less, 7 or less, 6 or less, 5 or less, 4 or less, 3 or less, 2 or less, or 1 or less.

(Anti-HMGBI antibody or antigen-binding fragment thereof)

The anti-HMGB1 antibody may be either a monoclonal antibody or a polyclonal antibody, but is preferably a monoclonal antibody from the viewpoint of uniform pharmaceutical quality. Examples of the monoclonal antibody include non-human animal-derived antibodies, human antibodies, humanized antibodies, or chimeric antibodies. Preferred is a human antibody, a humanized antibody, or a chimeric antibody. More preferred is a human antibody or a humanized antibody.

In the anti-HMGBI antibody, the full length or part of the antibody heavy and/or light chains, the entire or part of the constant regions, or the entire or part of the CDRs may be replaced by those of another anti-HMGBI antibody or those of another antibody that binds to an antigen other than HMGB1, as long as they have binding activity to their antigen, HMGB1.

HMGB1, which is an antigen recognized by the anti-HMGB1 antibody, may be HMGB1 derived from, for instance, a human or non-human mammal, but is preferably derived from the same species as a subject given the antibody-drug conjugate.

Examples of the isotype of the monoclonal antibody include IgG (e.g., IgG1, IgG2, IgG3, IgG4), IgM, IgA (e.g., IgA1, IgA2), IgD, or IgE. Preferred is IgG or IgM, and more preferred is IgG2.

The epitope of an anti-HMGB1 antibody used in an antibody-drug conjugate of the invention may be, for instance, a polypeptide within the C-terminal region or the B-Box region of HMGB1.

For instance, the epitope of the anti-human HMGB1 antibody preferably contains the amino acid sequence having EEEDDDDE (SEQ ID NO: 1) as an epitope within the C-terminal region or LKEKYEKDIA (SEQ ID NO: 2) as an epitope within the B-Box region.

The affinity of an anti-HMGB1 antibody for an HMGB1 epitope is evaluated, for instance, by a dissociation constant Kd obtained by measurement using surface plasmon resonance. Kd is preferably $1 \times 10^{-7}$ M or less and more preferably $1 \times 10^{-8}$ M or less.

Examples of the human HMGB1-specific monoclonal antibody include a humanized antibody #10-22 that recognizes the epitope set forth in SEQ ID NO: 1 as disclosed in WO2014/115430 or a humanized antibody #11-19 that recognizes the epitope having the amino acid sequence set forth in SEQ ID NO: 2.

The human HMGB1-specific monoclonal antibody is not limited to the combination of CDR sequences of the antibody that recognizes the epitope having the amino acid sequence set forth in SEQ ID NO: 1 or SEQ ID NO: 2. For instance, the amino acid sequence of the antibody may include a total of 6 CDR sequences of CDRH1 to 3 and CDRL1 to 3 with 9, 8, 7, 6, 5, 4, 3, 2, or 1 amino acid residue(s) deletion, substitution, insertion, or addition, or mutation caused by a combination of any two or more of them.

Further, the human HMGB1-specific monoclonal antibody is not limited to the combination of the heavy chain variable region and the light chain variable region in the humanized antibody that recognizes the epitope having the amino acid sequence set forth in SEQ ID NO: 1 or SEQ ID NO: 2. For instance, the amino acid sequence of the humanized antibody may include amino acid sequences of the heavy chain variable region and the light chain variable region with 9, 8, 7, 6, 5, 4, 3, 2, or 1 amino acid residue(s) deletion, substitution, insertion, or addition, or mutation caused by a combination of any two or more of them.

Alternatively, in the human HMGB1-specific monoclonal antibody, its heavy chain variable region and light chain variable region have 90% or higher, preferably 95% or higher, more preferably 98% or higher, and still more preferably 99% or higher homology to the heavy chain variable region and the light chain variable region, respectively, of the humanized antibody that recognizes the epitope having the amino acid sequence set forth in SEQ ID NO: 1 or SEQ ID NO: 2.

Alternatively, in the human HMGB1-specific monoclonal antibody, its entire FR has 90% or higher, preferably 95% or higher, more preferably 98% or higher, and still more preferably 99% or higher homology to the entire FR of the humanized antibody that recognizes the epitope having the amino acid sequence set forth in SEQ ID NO: 1 or SEQ ID NO: 2.

Throughout the specification, the amino acid sequence homology is represented by a numerical value of identity obtained by alignment under the default settings of the NCBI BLAST blastp suite.

The anti-HMGBI antibody used in an antibody-drug conjugate of the invention may be produced, for example, from a hybridoma derived from B lymphocytes obtained by immunizing an animal with the whole HMGB1 or an epitope-containing polypeptide. Examples of the specific procedure that can be used include the procedures disclosed in WO2007/049468, US2009/0252739, WO2014/115430, or FASEB J. 2007, Vol.21, pp.3904-3916.

Examples of the animal used for immunization include, but are not particularly limited to, warm-blooded animals, preferably rabbits, mice, rats, sheep, goats, cows, horses, guinea pigs, monkeys, dogs, cats, hamsters, or chickens.

In addition, an anti-HMGBI antibody or antigen-binding fragment thereof may be produced by a procedure including: introducing a polynucleotide containing a nucleotide sequence encoding all or part of the variable region(s) of the antibody into, for instance, an animal cell (e.g., a Chinese hamster ovary (CHO) cell, monkey kidney (COS) cell, HEK293 cell, NSO cell, HeLa cell, baby hamster kidney (BHK) cell, human hepatocarcinoma cell (e.g., HepG2)), a non-animal eukaryotic cell (e.g., an insect cell), a bacterial cell (e.g., *Escherichia coli*), yeast, an insect, a plant, a transgenic animal (e.g., a cow, chicken), or an *in vitro* translation system (e.g., a eukaryotic or E. coli-derived cell-free translation system); and expressing the antibody. For production of a rabbit-derived antibody, a mouse chimeric antibody, human chimeric antibody, humanized antibody, or antigen-binding fragment thereof, the aforesaid polynucleotide is preferably introduced to produce the antibody.

(Drug moiety)

A drug used for the drug moiety of an antibody-drug conjugate in the invention is not particularly limited and may be selected, if appropriate, depending on, for instance, a subject's disease. Further, the drug may be used singly or a plurality of kinds may be combined. Examples of the drug include one or two or more kinds selected from the group consisting of cytotoxic agents, cytoprotectants (e.g., statins, apoptosis inhibitors, neuroprotective agents, sulforaphane), probes, angiogenesis inhibitors, immunosuppressants, antihypertensive drugs, vasopressors, pain relievers, cytokines, antibiotics, and immune checkpoint inhibitors.

Examples of available chemical species of the drug include, natural organic compounds, synthetic organic compounds, proteins (e.g., antibodies, antibody fragments, enzymes), DNA, RNA, nucleic acid analogs (e.g., PNA, LNA, morpholino nucleic acid, S-modified oligos), peptides, nanoparticles, quantum dots, atoms (e.g., radionuclides), vesicles (e.g., PLGA nanoparticles), saccharides, or polysaccharides.

Examples of the cytotoxic agents include chemotherapeutic agents, radioisotopes, radiation sensitizers, near-infrared absorbers (e.g., IR700 (Nature Medicine, 2011, Vol. 17, pp. 1685-1691)), V-ATPase inhibitors, proapoptotic agents, Bcl2 inhibitors, HSP90 inhibitors, IAP inhibitors, MCL1 inhibitors, microtube stabilizers, microtube destabilizing agents, mTor inhibitors, auristatin, dolastatin, maytansine, mertansine, emtansine, duocarmycin, calicheamicin, trichothecene, diphtheria A chain, a non-binding active fragment of diphtheria toxin, exotoxin A chain (from *Pseudomonas aeruginosa*), ricin A chain, abrin A chain, modecine A chain, alpha-sarcin, Aleurites fordii proteins, dianthin proteins, Phytolacca americana proteins (PAPI, PAPII, and PAP-S), Momordica charantia inhibitors, crucine, crotin, Sapaonaria oficinalis inhibitors, geronine, mitogellin, restrictocin, phenomycin, enomycin and tricothecene CC1065, MetAP (methionine aminopeptidase), CRM1 nuclear transport inhibitors, DPPIV inhibitors, proteasome inhibitors, protein synthesis inhibitors, RNA polymerase inhibitors, DNA damaging agents, kinase inhibitors, CDK2 inhibitors, CDK9 inhibitors, kinesin inhibitors, HDAC inhibitors, DNA alkylating agents, DNA intercalating agents, DNA sub-groove binding agents, DHFR inhibitors, or anti-cancer antibiotics.

Examples of the chemotherapeutic agents include azaribine, anastrozole, azacytidine, bleomycin, bortezomib, bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxy urea, idarubicin, ifosfamide, leucovorin, lomustine, maytansinoid, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, taxol, testosterone propionate, thalidomide, thioguanine, thiotepa, teniposide, topotecan, uracil mustard, vinblastine, vinorelbine, vincristine, or trastuzumab.

Examples of the radioisotopes include iodine ($^{131}$I, $^{125}$I, $^{123}$I, and $^{121}$I) , carbon ($^{14}$C) , sulfur ($^{35}$S), tritium ($^{3}$H), indium ($^{115}$In, $^{113}$In, $^{112}$In, and $^{111}$In), technetium ($^{99}$Tc), thallium ($^{201}$Tl), gallium ($^{68}$Ga, $^{67}$Ga), palladium ($^{103}$Pd), molybdenum ($^{99}$Mo), xenon ($^{133}$Xe), fluorine ($^{18}$F), $^{153}$Sm, $^{177}$Lu, $^{159}$Gd, $^{149}$Pm, $^{140}$La, $^{175}$Yb, $^{166}$Ho, $^{90}$Y, $^{47}$Sc, $^{186}$Re, $^{188}$Re, $^{142}$Pr, $^{105}$Rh, $^{97}$Ru, $^{68}$Ge, $^{57}$Co, $^{65}$Zn, $^{85}$Sr, $^{32}$P, $^{153}$Gd, $^{159}$Yb, $^{51}$Cr, $^{54}$Mn, $^{75}$Se, $^{64}$Cu, $^{113}$Sn, or $^{117}$Sn.

Examples of the probes include, but are not particularly limited to, fluorescent substances, luminescent substances, radioactive probes (e.g., $^{3}$H, $^{14}$C, $^{15}$N, $^{35}$S, $^{90}$Y, $^{99}$Tc, $^{111}$In, $^{123}$I, $^{125}$I, $^{131}$I), nuclear magnetic resonance (NMR) imaging-use spin labels (e.g., $^{123}$I, $^{131}$I, $^{111}$In, $^{13}$C, $^{15}$N, $^{17}$O, $^{19}$F, Gd, Mn, Fe), positron-releasing substances, magnetic particles, fine particles (e.g., metal nanoparticles, plastic nanoparticles), contrast agents, enzymes (e.g., horseradish peroxidase, alkaline phosphatase, β-galactosidase, acetylcholine esterase, luciferase), biotin, streptavidin, or avidin.

Examples of the fluorescent substances include green fluorescent protein (GFP) or derivatives thereof, various phosphors of Alexa Fluor (registered trademark) (e.g., Alexa Fluor 430), or rhodamine-based, coumarin-based, oxazine-based, carbopyronine-based, cyanine-based, and pyrromethene-based, naphthalene-based, biphenyl-based, anthracene-based, phenanthrene-based, pyrene-based, carbazole-based, Cy-based, EvoBlue-based, fluorescein-based, or pyrrolopyridine-based phosphors, or pharmaceutically acceptable derivatives thereof.

Examples of the angiogenesis inhibitors include axitinib, thalidomide, angiostatin, endostatin, metastatin, anti-VEGF antibodies (e.g., bevacizumab), or VEGFR-2 inhibitors (e.g., SU5416, SU6668).

(Linker)

The linker used in the invention may be a linker that may be cleaved under conditions in which the compound or the antibody used herein retain their activity; cleavable linkers that receive a cleavage such as an acid-induced cleavage (e.g., hydrazone, acetal, cis-aconitate-like amide), which occurs under acidic conditions, for instance, in lysosome; a photo-induced cleavage; a peptidase-induced cleavage induced by such as a cathepsin or other lysosomal peptide cleavage enzyme; an esterase-induced cleavage; or a disulfide bond cleavage. Alternatively, the linker may be substantially resistant to any cleavage (e.g., a stable or non-cleavable linker). Alternatively, examples of the linker may include a procharge linker (see, for instance, US2009/0274713), a hydrophilic linker, or a dicarboxylic acid-based linker.

In one embodiment, the antibody-drug conjugate of the invention may be prepared using a cross-linking reagent with reactive functionality that allows the drug moiety to be conjugated to the antibody. For example, cysteine, thiol, or amine, or, for instance, the N-terminus or an amino acid side chain (e.g., a lysine side chain on the antibody or antigen binding fragment) can form a bond with a functional group of the cross-linking reagent. Then, the linker portion is constructed using all or part of the cross-linking reagent, or a plurality of the cross-linking reagents.

In another embodiment, the linker may function to react with free cysteine present on the antibody to form a covalent bond. Non-limiting examples of such reactive functionality include maleimides, haloacetamides, $\alpha$-haloacetyl, active esters such as succinimide ester, 4-nitrophenyl ester, pentafluorophenyl ester, and tetrafluorophenyl ester, anhydrides, acid chlorides, sulfonyl chlorides, isocyanates, or isothiocyanates.

In still another embodiment, the linker may function to react with an electrophilic group present on the antibody. Examples of such an electrophilic group include an aldehyde group or a ketone carbonyl group. In some embodiments, the reactive heteroatom functionality of the linker may react with an electrophilic group on the antibody to form a covalent bond to the antibody unit. Examples of such reactive functionality include hydrazide, oxime, amino, hydrazine, thiosemicarbazone, hydrazine carboxylate, or arylhydrazine.

In yet another embodiment, the linker is selected from the group consisting of bis-maleimide-trioxyethylene glycol (BMPEO), N-($\beta$-maleimidepropyloxy)-N-hydroxysuccinimide ester (BMPS), N-($\epsilon$-maleimide caproyloxy)succinimide ester (EMCS), N-[$\gamma$-maleimidebutyryloxy]succinimide ester (GMBS), 1,6-hexane-bis-vinylsulfone (HBVS), succinimidyl 4-(N-maleimidemethyl)cyclohexane-1-carboxy-(6-amidcaproic acid) (LC-SMCC), m-maleimidebenzoyl-N-hydroxysuccinimide ester (MBS), 4-(4-N-maleimidephenyl)butyrate hydrazide (MPBH), succinimidyl 3-(bromacetamide)propionate (SBAP), succinimidyl iodoacetate (SIA), succinimidyl(4-iodoacetyl)aminobenzoate (SIAB), N-succinimidyl-3-(2-pyridyldithio)propionate (SPDP), N-succinimidyl-4-(2-pyridylthio)pentanoate (SPP), succinimidyl 4-(N-maleimidemethyl)cyclohexane-1-carboxylate (SMCC), succinimidyl 4- (p-maleimidephenyl)butyrate (SMPB), succinimidyl 6-[$\beta$-maleimide propionamide)hexanoate] (SMPH), iminothiolane (IT), sulfo-EMCS, sulfo-GMBS, sulfo-KMUS, sulfo-MBS, sulfo-SIAB, sulfo-SMCC, sulfo-SMPB, and succinimidyl-(4-vinylsulfone) benzoate (SVSB).

Examples of such a linker-forming reagent include bis-maleimide reagents such as dithiobismaleimidoethane (DTME), 1,4-bis-maleimido butane (BMB), 1,4-bismaleimidyl-2,3-dihydroxybutane (BMDB), bis-maleimido hexane (BMH), mis(maleimido)ethane (BMOE), BM(PEG)$_2$ (shown below), and BM(PEG)$_3$ (shown below); bifunctional derivatives of imide esters (e.g., dimethyl adipimidate HC1), active esters (e.g., disuccinimidyl suberate), aldehydes (e.g., glutaraldehyde), bis-azido compounds (e.g., bis(p-azidobenzoyl)hexanediamine), bis-diazonium derivatives (e.g., bis-(p-diazonium benzoyl)-ethylenediamine), diisocyanates (e.g., toluene 2,6-diisocyanate), or bis-active fluorine compounds (e.g., 1,5-difluoro-2,4-dinitrobenzene).

In further embodiment, the linker comprises a polypeptide. Examples include, but are not limited to, dipeptides, tripeptides, tetrapeptides, or pentapeptides. The dipeptides are not limited and selected from the group consisting of valine-citrulline (vc or val-cit), alanine-phenylalanine (af or ala-phe); phenylalanine-lysine (fk or phe-lys); phenylalanine-homolysine (phe-homolys); and N-methyl-valine-citrulline (Me-val-cit). The tripeptides are not limited and selected from the group consisting of glycine-valine-citrulline (gly-val-cit) and glycine-glycine-glycine (gly-gly-gly).

The amino acid units of the polypeptide included in the linker may include naturally occurring amino acid residues and/or non-naturally occurring amino acid analogs such as citrulline and/or other amino acids. The amino acid units may be designed and optimized for enzymatic cleavage by specific enzymes such as tumor-related proteases, cathepsins B, C and D, or plasmin proteases.

These polypeptides may be synthesized by translating a fusion polypeptide including, as components, the antibody or antigen-binding fragment and the drug moiety.

The number of carbon atoms in the linker portion may be, for instance, 3 or more, 4 or more, 5 or more, 6 or more, 7 or more, 8 or more, 9 or more, 10 or more, 12 or more, or 15 or more, and for instance, 50 or less, 40 or less, 30 or less, or 20 or less.

[Pharmaceutical composition]

The pharmaceutical composition of the invention comprises the antibody-drug conjugate described in the above section [Antibody-drug conjugate] and a pharmaceutically acceptable carrier.

Examples of the dosage form of the pharmaceutical composition in the invention include liquid, semi-solid, paste, solid, or powder dosage forms such as solutions (e.g., injectable, enteral, or transfusionable solutions), microemulsions, dispersions, suspensions, tablets, powders, granules, capsules, troches, pills, liposomes, suppositories, poultices, or tapes. The preferred form varies depending on the intended dosage form, dose, frequency of administration, duration of administration, symptoms/age/sex/body weight of an administration object, and prevention/treatment purpose, and is a solution form feasible for injection, enteral administration, or infusion.

A preferable dosage form of the pharmaceutical composition of the invention is parenteral administration (e.g., intravenous, subcutaneous, intraperitoneal, intramuscular, or intragastrointestinal administration). In one embodiment, the pharmaceutical composition of the invention is administered by intravenous infusion, tube infusion, or intravenous injection. In another embodiment, the pharmaceutical composition of the invention is administered by intramuscular injection or subcutaneous injection.

The injectable preparation can be provided by dissolving the active ingredient in a liquid or freeze-drying the active ingredient in a flint or amber vial, ampule, plastic container, glass container, or prefilled syringe.

Generally speaking, the pharmaceutical composition of the invention is preferably sterile or stable under conditions during manufacture and storage. The pharmaceutical composition of the invention may be formulated in any of the above-mentioned forms, such as a solution, a microemulsion, a dispersion, or a liposome.

For instance, a sterile injectable solution may be prepared by mixing the required amount of the active ingredient (i.e., an antibody-drug conjugate of the invention) with one or a combination of the above ingredients in a suitable solvent, if necessary; and then performing sterilization treatment such as filtration sterilization.

For instance, the dispersion can be prepared using a powder formulation containing the active ingredient with a sterile vehicle containing a basic dispersion medium and other necessary ingredient(s) from those listed above. In addition, the powder formulation can be obtained, for example, by freeze-vacuum drying and spray drying of the above filtered sterile solution.

Examples of the pharmaceutically acceptable carrier include any or all of physiologically compatible solvents, dispersion media, coatings, isotonic agents, absorption enhancers, or absorption retarders. Also, examples of the pharmaceutically acceptable carrier include water; salt solutions; phosphate buffered saline (PBS); saccharides such as monosaccharides, disaccharides, oligosaccharides, polysaccharides (e.g., dextrin, dextran, isomaltodextrin, cellulose, pullulan, chitin, chitosan, guar gum, carrageenan) and derivatives thereof; or alcohols such as glycerol and ethanol. They can be used singly or in combination, if appropriate. In the case of use as an injection, it is possible to use a pH regulator or isotonic agent such as the above-mentioned saccharides, sugar alcohols such as mannitol, sorbitol, or maltitol, or sodium chloride which can be appropriately used alone or in combination.

Depending on the form of the pharmaceutical composition, the pharmaceutically acceptable carrier optionally further comprises auxiliary substances such as wetting agents, emulsifiers, preservatives, buffers, cryoprotectants, stabilizers, excipients, bulking agents, and/or pH regulators, in a suitable amount allowing for maintenance or increase in shelf life or effectiveness of the antibody or the antigen-binding fragment thereof.

It is possible to use, as the buffer, for instance, L-histidine (1 to 50 mM) at a pH of 5.0 to 7.0 (optimally pH of about 6) and 5 to 10 mM L-histidine in the optimal case. Examples of other suitable buffers include sodium succinate, sodium citrate, sodium phosphate, or potassium phosphate.

It is possible to use, as the cryoprotectant or excipient, for instance, 0 to 10% (optimally, 0.5 to 5%) of sugar such as glucose, fructose, sucrose, or maltose. Examples of other suitable cryoprotectants or excipients used include saccharides such as mannitol, maltitol, trehalose, or lactose.

It is possible to use, as the stabilizer, for instance, 1 to 50 mM (optimally, 5 to 10 mM) amino acids such as L-methionine. Examples of other suitable stabilizers include glycine, arginine, or polysorbate 80.

Examples of the bulking agents include, but are not particularly limited to, the above-mentioned saccharides or derivatives thereof, particularly, 1 to 10% trehalose, maltitol, or mannitol (optimally, 2 to 4%).

Since the pharmaceutical composition of the invention can deliver a drug to a living tissue including the above-described proangiogenic or hyperpermeable vasculature, the pharmaceutical composition is suitably used for proangiogenic disease or vascular hyperpermeability-related disease.

The term "proangiogenic disease" as used herein refers to a disease associated with increased angiogenesis. Examples of the proangiogenic disease include benign or malignant tumors (particularly solid tumors), wet age-related macular degeneration, macular edema, atherosclerosis, diabetic retinopathy, retrolental fibroplasia, hemangiomas, intraocular neovascular disease, proliferative retinopathy, angiogenic glaucoma, rheumatoid arthritis, or psoriasis.

Among them, the pharmaceutical composition of the invention is suitably applicable to benign or malignant solid tumors because the effect of inhibiting the nutrition or oxygen supply to surrounding tissues markedly exerted by angiogenesis suppression or neovascularity destruction.

If the pharmaceutical composition of the invention is applied to these proangiogenic diseases, it is preferable that the antibody-drug conjugate in the pharmaceutical composition contains at least a cytotoxic agent or an angiogenesis inhibitor as the drug.

Examples of the malignant tumors include malignant melanoma, squamous cell carcinoma, basal cell carcinoma, lung cancer (including small cell lung cancer, non-small cell lung cancer, lung adenocarcinoma, and squamous cell carcinoma of the lung), peritoneal cancer, hepatocellular carcinoma, gastric or stomach cancer (including gastrointestinal cancer), pancreatic cancer, glioma, cervical cancer, ovarian cancer, liver cancer, bladder cancer, liver cancer, breast cancer, colon cancer, colorectal cancer, endometrial cancer, uterine cancer, salivary adenocarcinoma, kidney or renal cancer, prostate cancer, pudendal cancer, thyroid cancer, eyelid tumors (including fat adenocarcinoma and basal cell cancer), conjunctival tumors, orbital tumors (including lacrimal gland tumors), intraocular tumors (including retinal blastoma and choroidal malignant melanoma), malignant lymphoma, ocular metastatic tumors, or various types of head and neck cancer (including oral cancer, pharyngeal cancer, epipharyngeal carcinoma, mesopharyngeal carcinoma, hypopharyngeal carcinoma, laryngeal cancer, nasal/sinus cancer, salivary adenocarcinoma, and thyroid cancer).

The term "vascular hyperpermeability-related disease" as used herein refers to a disease associated with increased vascular permeability. The vascular permeability is also increased by chronic or acute, strong or systemic inflammation. Thus, examples of the vascular hyperpermeability-related disease include epilepsy, stroke, cerebral infarction, cerebral vasospasm, traumatic brain injury, chronic inflammation, neurodegenerative diseases (e.g., Alzheimer's disease, Parkinson's disease, amyotrophic lateral sclerosis (ALS), Huntington's disease, spinocerebellar degeneration), acute inflammation, or sepsis.

Among them, the pharmaceutical composition of the invention is preferably applied to diseases associated with the BBB disruption. Examples of such diseases include epilepsy, stroke, cerebral infarction, cerebral vasospasm, traumatic brain injury, chronic inflammation, or neurodegenerative disease.

In the case of using the pharmaceutical composition of the invention for these vascular hyperpermeability-related diseases, it is preferable that the antibody-drug conjugate in the pharmaceutical composition contains at least a cytoprotectant as the drug.

The subject for the pharmaceutical composition of the invention is a human or non-human animal and preferably a human.

The pharmaceutical composition of the invention may be administered, for instance, daily, weekly, 1 to 7 times per week, once every 2 weeks, once every 3 weeks, once every 4 weeks, once every 5 weeks, once every 6 weeks, once every 7 weeks, or once every 8 weeks.

The dose of the pharmaceutical composition of the invention is not particularly limited, and may be changed, if appropriate, depending on, for instance, its composition, carrier, purpose of use, the age, body weight, and sex of the subject, and/or applicable disease. The dose of the pharmaceutical composition of the invention in terms of the mass of the antibody-drug conjugate included in the pharmaceutical composition may be, for instance, 0.0001 mg/kg (subject's body weight) or more, 0.001 mg/kg (patient's weight) or more, or 0.01 mg/kg (subject's weight) or more, and 200 mg/kg (patient's weight) or less, 100 mg/kg (subject's weight) or less, 20 mg/kg (subject's weight) or less, 10 mg/kg (subject's weight) or less, 5 mg/kg (subject's weight) or less, 2 mg/kg (subject's weight) or less, 1 mg/kg (subject's weight) or less, 0.5 mg/kg (subject's weight) or less, 0.2 mg/kg (subject's weight) or less, or 0.1 mg/kg (subject's weight) or less.

The pharmaceutical composition of the invention may be further used in combination with one or two or more kinds selected from the group consisting of additional antibodies or antibody-drug conjugates other than the anti-HMGB1 antibody or the antibody-drug conjugate of the invention, cytotoxic agents, cytoprotectants, probes, angiogenesis inhibitors, immunosuppressants, antihypertensive drugs, vasopressors, pain relievers, cytokines, antibiotics, and immune checkpoint inhibitors.

The pharmaceutical composition of the invention may be further used in combination with an irradiation of proton beam or heavy particle beam on the subject, or a photodynamic therapy (PDT) on the subject by using such as a gamma ray, an X-ray, or a light (e.g., near infrared light absorbed by IR700).

[Detection reagent]

The detection reagent of the invention comprises the antibody-drug conjugate described in the above section [Antibody-drug conjugate].

The detection reagent of the invention comprises a pharmaceutical used to obtain information about treatment, diagnosis or prevention, or a non-pharmaceutical such as a reagent used for research or the like.

In one embodiment, the drug moiety of the antibody-drug conjugate included in the detection reagent of the invention comprises at least a probe.

The subject for the detection reagent of the invention is an individual, organ, tissue, or cell of a human or non-human animal, and preferably a human individual. When used in a human individual, the detection reagent is administered preferably by intravenous infusion, intravenous injection, intramuscular injection, or subcutaneous injection, and more preferably by intravenous infusion or intravenous injection.

In one embodiment, during use of the detection reagent of the invention, a probe different from the antibody-drug conjugate may be further administered to the subject who receives the detection reagent.

In one embodiment, during use of the detection reagent of the invention, the subject who receives the detection reagent may be irradiated with, for instance, an X-ray, a light (including near infrared light), or a magnetic field (e.g., MRI), which may be used singly or in combination, if appropriate.

The detection reagent of the invention can be used, for instance, to obtain images of or information about the state of, for instance, proangiogenic or hyperpermeable vasculature or vascular endothelial cells.

The detection reagent of the invention is, for instance, used for diagnosis of proangiogenic disease or vascular hyperpermeability-related disease and preferably used for diagnosis of tumor. In certain embodiments, the detection reagent of the invention is used for diagnosis of melanoma.

Hereinafter, the invention will be described in more detail with reference to Examples. However, the invention is not limited to these Examples. About the molecular biological procedures used in the Examples, unless otherwise indicated, one can consult Molecular Cloning: A Laboratory Manual (Third Edition) (Sambrook et al., Cold Spring Harbor Laboratory Press, 2001).

EXAMPLES

[Reagents used]

(Antibodies, etc.)

(1) Anti-HMGB1 antibody

In the Examples, rat IgG was used as each anti-HMGB1 antibody. #10-22 rat IgG antibody (described in WO2014/115430) recognizing the epitope that is located in the C-terminal region of HMGB1 and has the amino acid sequence set forth in SEQ ID NO: 1; and #11-19 rat IgG antibody (described in FASEB J. 2007, Vol.21, pp.3904-3916) recognizing the epitope that is located in the B-box region of HMGB1 and has the amino acid sequence set forth in SEQ ID NO: 2 were used. The dissociation constant Kd for the rat antibody #10-22 or #11-19 against their respective epitopes was $1.5 \times 10^{-8}$ M or $3.0 \times 10^{-7}$ M, respectively. Note that the Kd was measured based on surface plasmon resonance.
Each antibody can be obtained from an antibody-producing hybridoma acquired by the procedure described in, for instance, WO2007/049468, US2009/0252739, WO2014/115430, or FASEB J. 2007, Vol.21, pp.3904-3916. The hybridoma acquisition procedure is overviewed below.

(a) Rat immunization

A mixture of commercially available bovine thymus-derived HMGB1 and HMGB2 (code number: 080-070741, manufactured by Wako Pure Chemical Industries, Ltd.) was administered, together with Freund's complete adjuvant, to the hind footpads of rats. Two weeks later, an increase in antibody titer was checked. Subsequently, after 5 weeks, lymph node cells were aseptically isolated from the swollen iliac lymph nodes.

(b) Cell fusion and cloning of anti-HMGB1 antibody-producing cell

The above iliac lymph node cells and mouse myeloma SP2/O-Ag14 (SP2) cells were fused using polyethylene glycol, and the resulting fused cells were cultured with a 96-well microplate. After primary screening using ELISA and secondary screening using Western blotting, anti-HMGB1 antibody-producing cells were cloned.

(2) Control antibody

In the Examples, an anti-KLH antibody was appropriately labeled or modified and then used as a control antibody. The anti-KLH antibody was prepared according to the procedure for producing an anti-HMGB1 antibody, except that keyhole limpet hemocyanin (KLH) (product number: H7017, manufactured by SIGMA-ALDRICH) was used as immunogen.

(3) Alexa Fluor 488-labeled antibody

The anti-HMGB1 antibody (1) and the anti-KLH antibody (2) were each labeled with Alexa Fluor 488 and then used. (4) Doxorubicin-conjugated antibody
The anti-HMGB1 antibody (1) or the anti-KLH antibody (2) was conjugated to a cytotoxic agent doxorubicin, of which the procedure was outsourced to Biologica Co., Japan. Specifically, an antibody-drug conjugate (Fig. 1A) was prepared by conjugating the amino group of a lysine side chain of the antibody to the amino group of doxorubicin while bridging them via an amide bond with a crosslinker glutaric acid. The drug/antibody ratio (DAR) of the doxorubicin-conjugated anti-HMGB1 antibody was 9.1, and the DAR of the doxorubicin-conjugated anti-KLH antibody was 8.2.

(5) Emtansine-conjugated antibody

Substantially the same procedure as in (4) was repeated to produce an anti-HMGB1 antibody or an anti-KLH antibody conjugated to emtansine (Fig. 1B) instead of the doxorubicin-linker moiety. The DAR of each emtansine-conjugated antibody was 0.9:1.

(Isolectin)

In order to specifically label vascular endothelial cells, isolectin, which is one of lectins, labeled with Alexa Fluor 594, which is a red fluorescent dye, was used (product number: 121413, manufactured by Invitrogen).

[Test Example 1: Verification of the uptake of anti-HMGB1 antibody into vascular endothelial cells]

Cultured proangiogenic endothelial cells EA.hy926 (CRL-2922, ATCC, Manassas, VA, USA) used for angiogenesis assay were used. EA.hy926 was suspended at $5 \times 10^5$ cells/mL in DMEM containing 10% fetal bovine serum (FCS) and 4 mM L-glutamine and inoculated to a 96-well microtiter plate. The cells were cultured at 37°C for 16 h and subjected to the assay in a monolayer confluent state.

Alexa Fluor 488-labeled anti-HMGB1 antibody (#10-22 or #11-19) or Alexa Fluor 488-labeled anti-KLH antibody as a control was added at a final concentration of 10 μg/mL to the above medium containing the confluent EA.hy926 cultured vascular endothelial cells. To further increase cell permeability, 100 ng/mL lipopolysaccharide (LPS) was added. Each nucleus was labeled with DAPI and the cell membrane was labeled with F-actin. Localization of the labeled antibody was observed 1, 2, and 4 h after the addition of these labeling reagents. A confocal laser scanning microscope (LMS780, manufactured by Carl Zeiss) was used for observation.

Figs. 2A to 2C show the results. As shown in Fig. 2A, the localization of the fluorescently labeled anti-HMGB1 antibody or the anti-KLH antibody (control antibody) is indicated by green fluorescence. It was found that the anti-HMGB1 antibody was taken up by cultured vascular endothelial cells after 1 h and accumulated in the form of granules around the nucleus over time. By contrast, since green fluorescence is hardly observed in the cells, the anti-KLH antibody is considered to be hardly incorporated into the cells.

Fig. 2B shows images comparing the cellular localizations of Alexa Fluor 488-labeled anti-HMGB1 antibodies with different epitopes. Any of the anti-HMGB1 antibodies with different epitopes was incorporated into cultured vascular endothelial cells.

Fig. 2C shows three-dimensionally reconstructed images of Fig. 2B. Granular fluorescently labeled anti-HMGB1 antibodies were present around the nucleus. This revealed that the anti-HMGB1 antibody-drug conjugate was incorporated into the cytoplasmic fraction, especially vesicles around the nucleus.

From the above results, it was concluded that the anti-HMGB1 antibodies can be taken up by proangiogenic or hyperpermeable vascular endothelial cells regardless of the difference in their epitopes.

[Test Example 2: Verification of the uptake of doxorubicin-conjugated anti-HMGB1 antibody into vascular endothelial cells]

The doxorubicin-conjugated rat anti-HMGB1 antibody (#10-22) or the doxorubicin-conjugated anti-KLH antibody (control antibody) was added at a final concentration of 10 μg/mL to a medium containing confluent EA.hy926 cultured vascular endothelial cells obtained by the same procedure as in Test Example 1. Further, 100 ng/mL of lipopolysaccharide (LPS) was added as a stimulant. Each nucleus was labeled with DAPI and the cell membrane was labeled with F-actin, and the localization of each labeled antibody was observed 4 h after the addition. A confocal laser scanning microscope (LMS780, manufactured by Carl Zeiss), was used for observation. The red autofluorescence of doxorubicin (excitation: 485 nm, emission: 590 nm) was used to detect each doxorubicin-conjugated antibody.

The results are shown in Figs. 3A and 3B. The doxorubicin-conjugated anti-HMGB1 antibody was incorporated into cultured vascular endothelial cells. By contrast, the anti-KLH antibody, which was a control antibody, was not incorporated into the cultured vascular endothelial cells even when stimulated with LPS.

The above results demonstrated that the anti-HMGB1 antibody conjugated to doxorubicin instead of the fluorescent label was also incorporated into the cultured vascular endothelial cells. Collectively, regardless of the kind of drug conjugated, it has been found that the anti-HMGB1 antibody-drug conjugate can be incorporated into proangiogenic or hyperpermeable vascular endothelial cells and the drug can thus be delivered to the cells.

[Test Example 3: Examination of whether anti-HMGB1 antibody accumulates specifically in tumor vascular endothelial cells]

First, $2.0 \times 10^5$ B16-BL6 melanoma cells were injected subcutaneously into the back of each mouse (C57BL/6J, 7-week-old female) and the resulting mice were reared in accordance with a conventional procedure to create melanoma-bearing mice. Next, 1 mg/kg (mouse body weight) of Alexa Fluor 488-labeled anti-HMGB1 antibody (#10-22) or Alexa Fluor 488-labeled anti-KLH antibody and 2 mg/kg (mouse body weight) of fluorescently labeled isolectin were administered into a tail vein of each tumor-bearing mouse at day 7 after the transplantation. After 30 min, the mice were sacrificed by blood removal and perfused with saline. After the perfusion, the melanoma tumor mass, liver, lung, and brain were excised, and frozen sections were prepared by a conventional procedure.

The sections thus obtained were observed under a confocal laser scanning microscope (LSM, manufactured by Carl Zeiss) at 200 × magnification.

LSM images of the melanoma tumor mass, liver, lung, and brain are shown in Figs. 4A to 4D, respectively. As shown in Fig. 4A, marked accumulation of the fluorescently labeled anti-HMGB1 was observed in tumor vascular endothelial cells in melanoma. By contrast, as shown in Figs. 4B to 4D, no accumulation of the fluorescently labeled anti-HMGB1 antibody was observed in the liver, lung, or brain vasculature, which were normal tissues.

The above results have revealed that the anti-HMGB1 antibody administered to the living body is specifically localized in the tumor vasculature. Then, when combined with the results of Test Example 1, it can be concluded that the localized anti-HMGB1 antibody was selectively taken up by tumor vascular endothelial cells.

[Test Example 4: *In vivo* test to check whether doxorubicin-conjugated anti-HMGB1 antibody exerts tumor-reducing effect]

First, $2.0 \times 10^5$ B16-BL6 melanoma cells were injected subcutaneously into the back of each mouse (C57BL/6J, 7-week-old female) and the resulting mice were reared in accordance with a conventional procedure to create melanoma-bearing mice. Day 6 after the transplantation, the tumor-bearing mice were divided into 4 groups each having 4 mice, and the following treatment groups (a) to (d) (the dose is the amount per mouse body weight, the group name is indicated in parentheses) were set. Each drug was administered into the tail veins of the mice. The amount of doxorubicin in (d) is the same as the amount of the doxorubicin moiety in (b) or (c).

(a) Phosphate buffered saline (PBS)
(b) 2.5 mg/kg of doxorubicin-conjugated rat anti-KLH antibody (a-KLHAb-Dox)
(c) 2.5 mg/kg of doxorubicin-conjugated rat anti-HMGB1 antibody (#10-22) ($\alpha$-HMGB1Ab-Dox)
(d) 98 μg/kg of free doxorubicin (Doxorubicin)

Day 7 after the administration, the same drug was readministered to the mice of each group. Then, day 11 after the first administration, 2 mg/kg (mouse body weight) of fluorescently labeled isolectin was administered into the tail vein. After 10 min, the mice were sacrificed by blood removal, perfused, and fixed, and tissue sections were then prepared. Fig. 5A provides an overview of the above test schedule.

The tumor volume of each mouse from the initiation of the first administration to day 11 after the administration was calculated by the following approximation formula based on the tumor diameter measured over time.

$$\text{Tumor volume } V = (W^2 \times L) / 2 \text{ (W: tumor minor diameter, L: tumor major diameter).}$$

The above perfused and fixed mouse melanoma tissue was paraffin-sectioned using a conventional procedure, and the proliferating cells were stained with an anti-Ki67 antibody (product number: ab15580, manufactured by abcam). The number of positive cells per visual field in the anti-Ki67 antibody-stained image was counted, and the number of Ki67-positive cells was compared among each group.

Further, apoptotic cells in the above tissue section were detected by the TUNEL protocol described below. For four individuals in each group, the number of cells and the number of TUNEL-positive cells in the four visual fields in the stained image of each individual were totaled to determine the percentage of TUNEL-positive cells.

<TUNEL protocol>

A commercially available In situ Apoptosis Detection Kit (manufactured by Takara Bio Inc.) was used to detect apoptotic cells by the following protocol.

(1) Prepare paraffin sections of melanoma obtained from each group (PBS group, $\alpha$-KLHAb-Dox group, $\alpha$-HMGB1Ab-Dox group, or Doxorubicin group).
(2) Deparaffinize the sections.
(3) Block endogenous peroxidase by 3% $H_2O_2$ solution for 5 min, and wash the sections with PBS.
(4) Place 50 μL (5 μL of TdT Enzyme + 45 μL of labeling buffer) of pre-prepared and ice-cooled labeling reaction solution on each slide, react them in a humid box at 37°C for 60 min, wash them with PBS, and react them for 5 min, and repeat, 3 times, the series of 5-min reaction steps.
(5) Observe each section under a fluorescence microscope.

Fig. 5B is a graph showing a change in the tumor volume measured and Fig. 5C shows a change in the body weight. In the α-HMGB1Ab-Dox group, the increase in the tumor volume was significantly suppressed when compared to the other groups. Meanwhile, there was no substantial difference in the body weight among the groups, and no serious adverse effects due to the administration were observed.

Fig. 5D shows anti-Ki67 antibody-stained images of the melanoma tissue, and Fig. 5E is a graph comparing the number of Ki67-positive cells. Tumor growth was more significantly suppressed in the α-HMGB1Ab-Dox group than in the other groups such as the Doxorubicin group.

Fig. 5F shows staining images of apoptotic cells in the tumor tissue, and Fig. 5G is a graph comparing the percentage of TUNEL-positive cells. Tumor cell apoptosis was induced at a significantly higher percentage in the α-HMGB1Ab-Dox group than in the α-KLHAb-Dox group and the Doxorubicin group. In the α-HMGB1Ab-Dox group, apoptotic cells spread concentrically around the blood vessel.

From the above results, it can be concluded that the doxorubicin-conjugated anti-HMGB1 antibody was taken up by vascular endothelial cells in the tumor to damage the cells by doxorubicin, resulting in suppression of tumor growth.

[Test Example 5: Verification of accumulation of anti-HMGB1 antibody in vascular endothelial cells at brain lesion]

First, 1 mg/kg (mouse body weight) of methylscopolamine was intraperitoneally injected and 350 mg/kg of pilocarpine was intravenously injected into each mouse (C57BL/6N, 5 females having a body weight of 18 to 23 g). This procedure was used to create pilocarpine-induced status epilepticus (PILO-SE) model mice.

Immediately after the induction of convulsions by administration of pilocarpine, 1 mg/kg of rat anti-HMGB1 antibody (#10-22) was intravenously injected. After 12 h, the brain was perfused and fixed with 4% paraformaldehyde to prepare frozen sections.

For the sections, nuclei were stained with DAPI and the anti-HMGB1 was stained with an FITC-labeled anti-rat IgG antibody. A confocal laser scanning microscope (LMS780, manufactured by Carl Zeiss) was used for observation.

Fig. 6 show the LSM images obtained. The central cavity represents a blood vessel. The anti-HMGB1 antibody (green) was localized around the blood vessel, suggesting that the antibody was localized in vascular endothelial cells.

It is known that increased brain vascular angiogenesis or permeability is observed due to brain disorders such as epilepsy, stroke, brain tumors, or neurodegenerative diseases. From the above results, it can be judged that the anti-HMGB1 antibody-drug conjugate specifically accumulates in proangiogenic or hyperpermeable vasculature caused by such brain disorders and is further incorporated into vascular endothelial cells.

[Test Example 6: *In vivo* test to check whether emtansine-conjugated anti-HMGB1 antibody exerts tumor-reducing effect]

First, $2.0 \times 10^5$ B16-BL6 melanoma cells were injected subcutaneously into the back of each mouse (C57BL/6J, 7-week-old female) and the resulting mice were reared in accordance with a conventional procedure to create melanoma-bearing mice. Day 9 after transplantation, the tumor-bearing mice were divided into 5 groups each having 5 mice, and the following treatment groups (a) to (e) (the dose is the amount per mouse body weight, the group name is indicated in parentheses) were set. Each drug was administered into the tail veins of the mice. The amount of emtansine in (e) is the same as the amount of the emtansine moiety in (a) or (b).

(a) 2.5 mg/kg of emtansine-conjugated rat anti-KLH antibody (α-KLHAb-DM1)
(b) 2.5 mg/kg of emtansine-conjugated rat anti-HMGB1 antibody (#10-22) (α-HMGB1Ab-DM1)
(c) 2.5 mg/kg of rat anti-KLH antibody (a-KLHAb)
(d) 2.5 mg/kg of rat anti-HMGB1 antibody (#10-22) (α-HMGB1Ab)
(e) 18 μg/kg of free emtansine (Emtansine)

Day 7 after administration, the same drug was readministered to the mice of each group. Then, day 10 after the first administration, 2 mg/kg (mouse body weight) of fluorescently labeled isolectin was administered to each mouse from the tail vein. After 10 min, the mice were sacrificed by blood removal, perfused, and fixed, and tissue sections were then prepared. Fig. 7B provides an overview of the above test schedule.

The tumor volume of each mouse from the initiation of the first administration to day 10 after the administration was determined by substantially the same procedure as in Test Example 4.

The test results show that the tumor volume at day 11 was smaller in the α-HMGB1Ab-DM1 group than in the other groups, and the highest tumor growth inhibitory effect was observed. In addition, the α-HMGB1Ab-DM1 group did not exhibit any decrease in mouse body weight. From the above, it can be concluded that an increase in the number of emtansine molecules conjugated to the antibody can further enhance the anti-tumor effects while suppressing adverse effects.

INDUSTRIAL APPLICABILITY

The conjugate of an anti-HMGB1 antibody or an antigen-binding fragment thereof and a drug (antibody-drug conjugate) according to the invention makes it possible to deliver the desired drug selectively to proangiogenic or hyperpermeable vascular endothelial cells. In addition, the antibody-drug conjugate is characterized in that the conjugate is not substantially incorporated into normal vasculature. Hence, the antibody-drug conjugate can effectively exert the desired pharmacological action of the drug, so that adverse effects caused by the drug can be avoided or reduced, thereby providing the benefit of reducing the burden on a patient. The industrial usefulness of the invention is therefore immeasurable.

**Claims**

1. An antibody-drug conjugate comprising:

   an antibody specifically binding to HMGB1 or an antigen-binding fragment thereof; and
   a drug moiety conjugated to the antibody or the antigen-binding fragment thereof.

2. The antibody-drug conjugate according to claim 1, wherein the drug moiety and the antibody or the antigen-binding fragment thereof are conjugated via at least one linker.

3. The antibody-drug conjugate according to claim 1 or 2, wherein the drug moiety comprises one or two or more members selected from the group consisting of cytotoxic agents, cytoprotectants, probes, angiogenesis inhibitors, immunosuppressants, antihypertensive drugs, vasopressors, pain relievers, cytokines, antibiotics, and immune checkpoint inhibitors.

4. The antibody-drug conjugate according to any one of claims 1 to 3, which is represented by formula (I):

$$A\text{-}(L\text{-}(D)_m)_n \qquad (I)$$

wherein, A is the antibody specifically binding to HMGB1 or the antigen-binding fragment thereof;
L is a linker;
D is the drug moiety;
m is an integer of 1 to 8; and
n is an integer of 1 to 20.

5. The antibody-drug conjugate according to any one of claims 1 to 4, wherein the antibody or the antigen-binding fragment thereof specifically binds to human HMGB1 and is a chimeric antibody, a humanized antibody, or a human antibody, or a fragment thereof.

6. The antibody-drug conjugate according to any one of claims 1 to 5, wherein the antibody is a monoclonal antibody.

7. The antibody-drug conjugate according to any one of claims 1 to 6, wherein the antibody or the antigen-binding fragment thereof specifically binds to a human HMGB1 epitope comprising an amino acid sequence set forth in SEQ ID NO: 1 or SEQ ID NO: 2.

8. The antibody-drug conjugate according to any one of claims 2 to 7, wherein the linker is selected from the group consisting of cleavable linkers, non-cleavable linkers, hydrophilic linkers, procharge linkers, and dicarboxylic acid-based linkers.

9. A pharmaceutical composition comprising the antibody-drug conjugate according to any one of claims 1 to 8 and a pharmaceutically acceptable carrier.

10. The pharmaceutical composition according to claim 9, which is used for treatment of a proangiogenic disease or a vascular hyperpermeability-related disease.

11. The pharmaceutical composition according to claim 9, which is used for treatment of:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or
(b) epilepsy, stroke, cerebral infarction, cerebral vasospasm, traumatic brain injury, chronic inflammation, neurodegenerative diseases, acute inflammation, or sepsis.

12. The pharmaceutical composition according to any one of claims 9 to 11, which is used in combination with one or two or more members selected from the group consisting of other antibodies, other antibody-drug conjugates, cytotoxic agents, cytoprotectants, probes, angiogenesis inhibitors, immunosuppressants, antihypertensive drugs, vasopressors, pain relievers, cytokines, antibiotics, and immune checkpoint inhibitors.

13. A detection reagent comprising the antibody-drug conjugate according to any one of claims 1 to 8.

14. The detection reagent according to claim 13, wherein the drug moiety comprises at least a probe.

15. Use of an antibody specifically binding to HMGB1 or an antigen-binding fragment thereof for drug delivery to a cell.

16. The use according to claim 15, wherein the cell is a vascular endothelial cell.

17. The use according to claim 15 or 16, comprising treating the cell with the antibody-drug conjugate according to any one of claims 1 to 8.

18. A method for treating or diagnosing a proangiogenic disease or a vascular hyperpermeability-related disease, said method comprising administering, to a subject in need thereof, the antibody-drug conjugate according to any one of claims 1 to 8.

19. A method for treating or diagnosing:

(a) benign or malignant tumors, wet age-related macular degeneration, macular edema, atherosclerosis, diabetic retinopathy, retrolental fibroplasia, hemangiomas, intraocular neovascular diseases, proliferative retinopathy, neovascular glaucoma, rheumatoid arthritis, or psoriasis; or
(b) epilepsy, stroke, cerebral infarction, cerebral vasospasm, traumatic brain injury, chronic inflammation, neurodegenerative diseases, acute inflammation, or sepsis;

said method comprising administering, to a subject in need thereof, the antibody-drug conjugate according to any one of claims 1 to 8.

20. The method according to claim 18 or 19, further comprising administering, to the subject, one or two or more members selected from the group consisting of other antibodies, other antibody-drug conjugates, cytotoxic agents, cytoprotectants, probes, angiogenesis inhibitors, immunosuppressants, antihypertensive drugs, vasopressors, pain relievers, cytokines, antibiotics, and immune checkpoint inhibitors.

21. The method according to any one of claims 18 to 20, further comprising irradiating the subject with a proton beam, a heavy particle beam, a gamma ray, an X-ray, or a light.

# Fig. 1A

Lysine side chain of antibody

# Fig. 1B

Lysine side chain of antibody

## Fig. 2A

### Anti-HMGB1 antibody (#10-22)

### Anti-KLH antibody (control antibody)

1h          2h          4h (Time)

Fig. 2B

Anti-KLH antibody

Anti-HMGB1 antibody
(#11-19)

Anti-HMGB1 antibody
(#10-22)

Fig. 2C

Fig. 3A

Fig. 3B

DAPI
(Cell nuclei)

Doxorubicin-conjugated
anti-HMGB1 antibody

Superposed

# Fig. 4A

Fig. 4B

## Fig. 4C

Fig. 4D

Superposed

Vascular endothelial cells (isolectin staining)

Antibody (Alexa Fluor 488)

Alexa Fluor 488-labeled anti-KLH antibody (control antibody)

Alexa Fluor 488-labeled anti-HMGB-1 antibody (#10-22)

Fig. 5A

C57BL/6J, 7weeks, ♀

Subcutaneously injection
B16-BL6 Melanoma,
2.0 x $10^5$ cells

1st treatment (intravenously injection)
1, PBS
2, α-KLHAb-Dox (2.5mg/kg)
3, α-HMGB1 Ab-Dox (2.5mg/kg)
4, Doxorubicin (98 μg/kg)

2nd reatment (intravenously injection)
The same treatment as in 1st treatment

Monitoring tumor volume & body weight

Day-6

Day0

Day7

Day11

10 minutes before sacrifice,
mice were intravenously treated
with isolectin Alexa Fluor 594
conjugate (2 mg / kg).

# Fig. 5B

Legend:
- PBS
- α-KLHAb-Dox
- α-HMGB1Ab-Dox
- Doxorubicin

Y-axis: Tumor volume(mm$^3$)

X-axis: Days after first treatment

Fig. 5C

Fig. 5D

Ki67 staining

PBS | α-KLHAb-Dox | α-HMGB1Ab-Dox | Doxorubicin

Fig. 5E

Fig. 5F

## Fig. 5G

Fig. 6

Fig. 7

EP 3 875 118 A1

1st treatment (intravenously injection)
1, α-KLHAb-DM1  (2.5mg/kg)
2, α-HMGB1 Ab-DM1  (2.5mg/kg)
3, α-KLHAb (2.5mg/kg)
4, α-HMGB1 Ab (2.5mg/kg)
5, Emtansine (18 µg/kg)

2nd reatment (intravenously injection)
The same treatment as in 1st treatment

**C57BL/6J, 7weeks, ♀**

Subcutaneously injection
B16-BL6 Melanoma,
2.0 x $10^5$ cells

Monitoring tumor volume & body weight

**Day-6**

**Day0**

**Day7**

**Day11**

10 minutes before sacrifice,
mice were intravenously treated
with isolectin Alexa Fluor 488
conjugate (2 mg / kg).

## INTERNATIONAL SEARCH REPORT

| International application No. |
|---|
| PCT/JP2020/047157 |

A. CLASSIFICATION OF SUBJECT MATTER
A61K 47/68(2017.01)i; A61K 39/395(2006.01)i; A61P 3/10(2006.01)i; A61P 9/00(2006.01)i; A61P 9/10(2006.01)i; A61P 17/02(2006.01)i; A61P 17/06(2006.01)i; A61P 25/00(2006.01)i; A61P 25/08(2006.01)i; A61P 27/02(2006.01)i; A61P 27/06(2006.01)i; A61P 29/00(2006.01)i; A61P 35/00(2006.01)i; C07K 16/18(2006.01)i
FI: A61K47/68; A61P3/10; A61P27/02; A61P35/00; A61P9/00; A61P27/06; A61P9/10; A61P25/00; A61P29/00; A61P29/00 101; A61P17/02; A61P17/06; A61P25/08; C07K16/18 ZNA; A61K39/395 N

According to International Patent Classification (IPC) or to both national classification and IPC

B. FIELDS SEARCHED

Minimum documentation searched (classification system followed by classification symbols)
A61K47/68; A61K39/395; A61P3/10; A61P9/00; A61P9/10; A61P17/02; A61P17/06; A61P25/00; A61P25/08; A61P27/02; A61P27/06; A61P29/00; A61P35/00; C07K16/18

Documentation searched other than minimum documentation to the extent that such documents are included in the fields searched
Published examined utility model applications of Japan     1922-1996
Published unexamined utility model applications of Japan     1971-2021
Registered utility model specifications of Japan     1996-2021
Published registered utility model applications of Japan     1994-2021

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | US 2014/0127134 A1 (YANG, H.) 08 May 2014 (2014-05-08) claim 1, paragraphs [0012], [0017], [0051], [0071], [0122]-[0123], [0127], [0171]-[0172] | 1-3, 5-15, 17-21 |
| Y | | 4 |
| A | | 16 |
| Y | JP 2016-518332 A (NOVARTIS AG) 23 June 2016 (2016-06-23) claim 1 | 4 |
| A | JP 2006-517537 A (ALCEDO BIOTECH GMBH) 27 July 2006 (2006-07-27) | 1-21 |

☐ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 February 2021 (24.02.2021) | 23 March 2021 (23.03.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| International application No. |
|---|
| PCT/JP2020/047157 |

| Patent Documents referred in the Report | Publication Date | Patent Family | Publication Date |
|---|---|---|---|
| US 2014/0127134 A1 | 08 May 2014 | WO 2012/170742 A2 | |
| JP 2016-518332 A | 23 Jun. 2016 | US 2014/0301946 A1 claim 1 WO 2014/160160 A2 EP 2968592 A2 KR 10-2015-0130333 A CN 105188763 A | |
| JP 2006-517537 A | 27 Jul. 2006 | US 2007/0154529 A1 WO 2004/061456 A2 EP 1579221 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014115430 A
- WO 2007049468 A
- US 20090252739 A
- US 20090274713 A

**Non-patent literature cited in the description**

- *Annual Review of Immunology,* 2011, vol. 29, 139-162
- *Nihon Yakurigaku Zasshi,* 2018, vol. 151, 4-8
- *Journal of Pharmacological Science,* 2019, vol. 140, 94-101
- **KABAT, E.A. et al.** Sequences of Proteins of Immunological Interest. Public Health Service, National Institutes of Health, 1991
- **HUDSON, PJ et al.** *Nat. Med.,* 2003, vol. 9, 129-134
- **PLUECKTHUN, A.** The Pharmacology of Monoclonal Antibodies. Springer-Verlag, 1994, vol. 113, 269-315
- *Nature,* 1989, vol. 341, 544-546
- *FASEB J.,* 2007, vol. 21, 3904-3916
- *Nature Medicine,* 2011, vol. 17, 1685-1691
- **SAMBROOK et al.** Molecular Cloning: A Laboratory Manual. Cold Spring Harbor Laboratory Press, 2001

```
                        SEQUENCE LISTING

    <110>   OKAYAMA UNIVERSITY
            SOWAKAI MEDICAL FOUNDATION

    <120>   ANTIBODY-DRUG CONJUGATES AND USE OF ANTIBODIES FOR DRUG DELIVERY

    <130>   PCT20075MD

    <150>   JP 2019-228542
    <151>   2019-12-18

    <160>   2

    <170>   PatentIn version 3.5

    <210>   1
    <211>   8
    <212>   PRT
    <213>   Homo sapiens

    <400>   1

    Glu Glu Glu Asp Asp Asp Asp Glu
    1               5


    <210>   2
    <211>   10
    <212>   PRT
    <213>   Homo sapiens

    <400>   2

    Leu Lys Glu Lys Tyr Glu Lys Asp Ile Ala
    1               5               10
```